# EUROPEAN PATENT SPECIFICATION

(11) **EP 4 288 562 B1**
(45) Date of publication and mention of the grant of the patent: **20.11.2024**
(21) Application number: 22706451.6
(22) Date of filing: 03.02.2022
(51) Int. Cl.: C12Q 1/6806

(54) **LONG INDEXED-LINKED READ GENERATION ON TRANSPOSOME BOUND BEADS**
ERZEUGUNG VON LANGEM INDEXIERTEM VERKNÜPFTEM LESEN AUF TRANSPOSOMGEBUNDENEN KÜGELCHEN
GÉNÉRATION DE LECTURES LONGUES INDEXÉES-LIÉES SUR DES BILLES LIÉES PAR TRANSPOSOMES

(30) Priority: 04.02.2021 US 202163145902 P
(43) Date of publication of application: 13.12.2023
(73) Proprietor: Illumina, Inc., San Diego, CA 92122 (US)
(72) Inventor: CHRISTIANSEN, Lena, San Diego, California 92122 (US); STEEMERS, Frank J., San Diego, California 92122 (US); SCHROTH, Gary, San Diego, California 92122 (US); THOMAS, Jerushah, San Diego, California 92122 (US); POKHOLOK, Dmitry K., San Diego, California 92122 (US)
(74) Representative: Witte, Weller & Partner Patentanwälte mbB
(86) International application number: PCT/US2022/015113
(87) International publication number: WO 2022/169972

(56) References cited:
- WO-A1-2018/118971
- WO-A1-2018/172726
- WO-A1-2019/217452
- WO-A1-2020/144373
- WO-A1-2020/165433
- WO-A1-2020/167866
- WO-A1-2022/010965
- WO-A1-2022/031955
- WO-A2-2014/145820
- WO-A2-2020/112604
- WO-A2-2021/154648
- CHEN HE ET AL: "Tagmentation on Microbeads: Restore Long-Range DNA Sequence Information Using Next Generation Sequencing with Library Prepared by Surface-Immobilized Transposomes", APPLIED MATERIALS & INTERFACES, vol. 10, no. 14, 15 March 2018 (2018-03-15), US, pages 11539 - 11545, XP055915782, ISSN: 1944-8244, Retrieved from the Internet <URL:https://pubs.acs.org/doi/pdf/10.1021/acsami.8b01560> DOI: 10.1021/acsami.8b01560
- FAN ZHANG ET AL: "Haplotype phasing of whole human genomes using bead-based barcode partitioning in a single tube", NATURE BIOTECHNOLOGY, vol. 35, no. 9, 26 June 2017 (2017-06-26), New York, pages 852 - 857, XP055584000, ISSN: 1087-0156, DOI: 10.1038/nbt.3897

## Description

### CROSS-REFERENCE TO RELATED APPLICATIONS

This application claims priority to U.S. Prov. App. No. 63/145,902 filed February 4, 2021 entitled "LONG INDEXED-LINKED READ GENERATION ON TRANSPOSOME BOUND BEADS".

### FIELD

Systems, methods, and compositions provided herein relate to compositions, systems, and methods for spatial index sequencing and nucleic acid library preparation.

### BACKGROUND

The detection of specific nucleic acid sequences present in a biological sample has been used, for example, as a method for identifying and classifying microorganisms, diagnosing infectious diseases, detecting and characterizing genetic abnormalities, identifying genetic changes associated with cancer, studying genetic susceptibility to disease, and measuring response to various types of treatment. A common technique for detecting specific nucleic acid sequences in a biological sample is nucleic acid sequencing.

Next generation sequencers are powerful tools that generate large amounts of genomic data per sequencing run. Interpreting and analyzing this large amount of data can be challenging. WO2018/118971 discloses tagmentation followed by partitioning into microdroplets comprising primer beads.

### SUMMARY

The present disclosure is related to systems, methods, and compositions for making indexed-linked reads using bead-bound transposomes and a droplet generator.

The invention provided herein relates to a system for nucleic acid indexed amplification, comprising: a plurality of contiguity beads, an indexed primer pool, and a detector for obtaining sequencing data. Each contiguity bead is associated with a transposome and each contiguity bead includes a bead-bound nucleic acid molecule. The indexed primer pool includes a plurality of primer beads and a solution primer. Each primer bead includes an adapter, a barcode, and a primer. The contiguity beads and primer beads are partitioned together within droplets. In some embodiments, the primer is a P5 primer. In some embodiments, the solution primer comprises adapters and primers. In some embodiments, the solution primer comprises B15 adapters and P7 primers. In some embodiments, the transposome comprises transposase and transposon.

In some embodiments, the contiguity beads and/or the primer beads are hydrogel beads include a hydrogel polymer and a crosslinker. In some embodiments, the hydrogel polymer includes polyethylene glycol (PEG)-thiol/PEG-acrylate, acrylamide/N,N'-bis(acryloyl)cystamine (BACy), PEG/polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, or collagen. In some embodiments, the crosslinker includes bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate. In some embodiments, the nucleic acid is a DNA molecule of 50,000 base pairs or greater.

The invention provided herein further relates to flow cell devices for nucleic acid indexed amplification. The device includes a solid support that includes a plurality of partitioned droplets. The plurality of partitioned droplets includes a contiguity bead associated with a transposomes, and including a bead-bound nucleic acid molecule and a primer bead comprising an adapter, a barcode, and a primer. The plurality of partitioned droplets are distributed along a surface of the solid support.

In some embodiments, the solid support is functionalized with a surface polymer. In some embodiments, the surface polymer is poly(N-(5-azidoacetamidylpentyl) acrylamide-co-acrylamide) (PAZAM) or silane free acrylamide (SFA). In some embodiments, the flow cell includes a patterned surface. In some embodiments, the patterned surface comprises wells. In some embodiments, the wells are from about 10 µm to about 50 µm in diameter, such as 10 µm, 15 µm, 20 µm, 25 µm, 30 µm, 35 µm, 40 µm, 45 µm, or 50 µm in diameter, or within a range defined by any two of the aforementioned values, and wherein the wells are about 0.5 µm to about 1 µm in depth, such as 0.5 µm, 0.6 µm, 0.7 µm, 0.8 µm, 0.9 µm, or 1 µm in depth, or within a range defined by any two of the aforementioned values. In some embodiments, the wells include a hydrophobic material. In some embodiments, the hydrophobic material comprises an amorphous fluoropolymer, such as CYTOP, Fluoropel^{®}, or Teflon^{®}. In some embodiments, the nucleic acid is a DNA molecule of 50,000 base pairs or greater. In some embodiments, the transposome comprises transposase and transposon.

The invention provided herein further relates to a method of nucleic acid indexing. The methods includes generating a plurality of contiguity beads for on bead tagmentation, each bead linked to a transposome, and comprising a bead-bound nucleic acid molecule, performing a tagmentation reaction on the nucleic acid molecule, generating a plurality of primer beads, each primer bead comprising an adapter, a barcode, and a primer, partitioning the contiguity beads and the primer beads together within droplets with a solution primer, amplifying nucleic acid molecule within the partitioned droplets, and indexing the nucleic acid molecule in each droplet.

In some embodiments, the nucleic acid is a DNA molecule of 50,000 base pairs or greater. In some embodiments, the methods further include performing nucleic acid amplification on nucleic acid molecule prior to performing the tagmentation reaction. In some embodiments, the amplification reaction comprises multiple displacement amplification (MDA). In some embodiments, the tagmentation reaction comprises contacting the nucleic acid with a transposase mixture comprising adapter sequences and transposomes. In some embodiments, the indexing is performed by polymerase chain reaction (PCR). In some embodiments, the droplets are partitioned into more than 900,000 different indexed PCR compartments. In some embodiments, the methods further include partitioning the droplets on a solid support. In some embodiments, the solid support is a flow cell device.

### BRIEF DESCRIPTION OF THE DRAWINGS

FIG. 1 is a schematic diagram of an aspect of a microfluidic droplet generator system that can be used to generate partitioned droplets of on bead-transposomes.
FIG. 2 depicts a schematic representation for exemplary methods for performing linked long read indexing, including contiguity-preserving transposition sequencing (CPT-seq) on beads (step 1), partition/indexed PCR (step 2), and indexed-linked reads (step 3).
FIG. 3 depicts a schematic representation for exemplary methods for performing long read indexing, including CPT-seq on beads, droplet partitioning, and indexed primer pool indexing.
FIG. 4 depicts a schematic representation of results of chromosome level phasing using the methods described herein.
FIG. 5 depicts results of number of islands compared to island length using the long read indexing methods described herein.
FIG. 6 depicts results of variant calling and phasing blocks using the long read indexing methods described herein (left) compared to 10X sequencing (right).
FIG. 7 depicts results of methods of long read indexing methods described herein performed on a human leukocyte antigen (HLA) region.
FIGs. 8A and 8B depict results of methods of long read indexing methods described herein performed on HLA-DPA1 (FIG. 8A) and HLA-A (FIG. 8B) region.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made. It will be readily understood that the aspects of the present disclosure, as generally described herein, and illustrated in the Figures, can be arranged, substituted, combined, separated, and designed in a wide variety of different configurations.

Aspects provided herein relate to long read indexing systems, devices, and methods. The systems include on bead tagmentation. The beads may include any of the beads disclosed herein, and having a transposome attached thereto, with nucleic acid molecules bound to the beads.

In some aspectss, the beads include hydrogel polymers and crosslinkers that are mixed in the presence of a transposome, and which form beads bound to transposome. In some aspects, the beads are prepared and later mixed with transposome, which are then bound to the beads. In some aspects, the beads are prepared in the presence of nucleic acid molecules, which wrap around or associate with the beads. In some aspects, the beads are first prepared, mixed with transposome, and then mixed with nucleic acid molecules. In some aspects, the beads enable co-assays to be performed on the same sample while maintaining contiguity. In particular, the methods, systems, and compositions provided herein allow confining and accessing biomolecules bound to the beads. Accordingly, in some aspects, the beads described herein are referred to herein as contiguity particles. Thus, the term "contiguity particle" as used herein refers to a bead used for contiguity-preserving transposition sequencing (CPT-seq).

The contiguity particles described herein may be used for next generation compartmentalization approaches and allow multi-analyte assays performed on nucleic acid molecules. The contiguity particles and methods of use described herein efficiently allow millions of nucleic acid molecules to be analyzed individually thereby reducing the cost of sample preparation and maintaining sample contiguity. The compositions and methods described herein maintain contiguity without the use of external compartmentalization strategies (microfluidics) such as emulsions, immobilization, or other micro-compartments.

In some aspects, the contiguity particles as described herein can be used in assays to analyze a nucleic acid molecule of interest. Assays that may be performed on the nucleic acid molecule may include, for example, DNA analysis, RNA analysis, nucleic acid sequencing, tagmentation, nucleic acid amplification, DNA library preparation, assay for transposase accessible chromatic using sequencing (ATAC-seq), contiguity-preserving transposition sequencing (CPT-seq), or any combination thereof performed sequentially.

The use of contiguity particles for performing one or more assays on a nucleic acid molecule may be used simultaneously on multiple contiguity particles in order to simultaneously perform co-assays on a number of nucleic acid molecules, for example from 10,000 to 1 million nucleic acid molecules, such as 10,000, 50,000, 100,000, 500,000, or 1 million nucleic acid molecules.

In some aspects, the methods described herein include methods for making indexed-linked reads for a variety of applications, including phasing and assembly. In some aspects, the method include combining on bead tagmentation with droplet indexing. In some aspects, droplet indexing includes any physical compartment indexing, including emulsions or plates. In some aspects, the beads provided herein include transposomes, which allow for performance of on bead tagmentation on nucleic acid molecules. In some aspects, each nucleic acid molecule wraps around the bead, generating bead bound fragments of the nucleic acid molecule. In some aspects, the methods are combined with indexing. In some aspects, the methods include partitioning the beads in droplets. In some aspects, the methods include performing indexed PCR in each droplet. In some aspects, each fragment from an individual nucleic acid molecule receives the same barcode, thereby generating indexed-linked reads.

Aspects of the methods, systems, and devices described herein have numerous advantages over prior methods. For example, the methods, systems, and devices described herein provides for controlled insert size and transfers the DNA without fragmenting DNA to physical partitions where they are uniquely indexed by PCR. In addition, CPT-seq on bead may be performed on more than 900,000 different indexed PCR compartments. Further, the CPT-seq on beads results in improved control of library insert (transposome density), increased efficiency of DNA transfer to droplet, robust DNA preparation, steps of the assay that may be performed prior to droplet (including, for example, Tn5 removal), and less DNA fragmentation. Aspects of the methods allow for template elution of beads, which results in free biotin elution of tagmented products in the droplets after heating. In addition, the enzymes associated with the methods provide for high amplification with strand displacing polymerase and increased amounts of enzyme. Finally, aspects of the methods provided herein result in increased DNA quality and allow for compatibility with lysate.

The methods, systems, and devices provided herein combine transposition on beads with transfer of tagmented nucleic acids to physical partitions. In some aspects, long indexed read production includes generation of bead bound transposomes and a droplet generator. In some aspects, the droplet generator includes a microfluidic device or emulsion. In some aspects, transposition on beads includes nucleic acid tagmentation and frequency, which may be controlled by the density of transposome on beads. In some aspects, beads are used to transfer tagmented nucleic acids to physical partitions.

As used herein, the term "reagent" describes an agent or a mixture of two or more agents useful for reacting with, interacting with, diluting, or adding to a sample, and may include agents used in assays described herein, including agents for lysis, nucleic acid analysis, nucleic acid amplification reactions, protein analysis, tagmentation reactions, ATAC-seq, CPT-seq, or SCI-seq reactions, or other assays. Thus, reagents may include, for example, buffers, chemicals, enzymes, polymerase, primers having a size of less than 50 base pairs, template nucleic acids, nucleotides, labels, dyes, or nucleases. In some aspects, the reagent includes lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

### Contiguity Particles

In some aspects, the bead has a polymer shell that was prepared from a hydrogel composition. As used herein, the term "hydrogel" refers to a substance formed when an organic polymer (natural or synthetic) is cross-linked via covalent, ionic, or hydrogen bonds to create a three-dimensional open-lattice structure that entraps water molecules to form a gel. In some aspects, the hydrogel may be a biocompatible hydrogel. As used herein, the term "biocompatible hydrogel" refers to a polymer that forms a gel that is not toxic to biological materials. In some aspects, the hydrogel material includes alginate, acrylamide, or polyethylene glycol (PEG), PEG-acrylate, PEG-amine, PEG-carboxylate, PEG-dithiol, PEG-epoxide, PEG-isocyanate, PEG-maleimide, polyacrylic acid (PAA), poly(methyl methacrylate) (PMMA), polystyrene (PS), polystyrene sulfonate (PSS), polyvinylpyrrolidone (PVPON), N,N'-bis(acryloyl)cystamine, polypropylene oxide (PPO), poly(hydroxyethyl methacrylate) (PHEMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations or mixtures thereof. In some aspectss, the hydrogel is an alginate, acrylamide, or PEG based material. In some aspects, the hydrogel is a PEG based material with acrylate-dithiol, epoxide-amine reaction chemistries. In some aspects, the hydrogel forms a polymer shell that includes PEG-maleimide/dithiol oil, PEG-epoxide/amine oil, PEG-epoxide/PEG-amine, or PEG-dithiol/PEG-acrylate. In some aspects, the hydrogel material is selected in order to avoid generation of free radicals that have the potential to damage intracellular biomolecules. In some aspects, the hydrogel polymer includes 60-90% fluid, such as water, and 10-30% polymer. In certain aspects, the water content of hydrogel is about 70-80%. As used herein, the term "about" or "approximately", when modifying a numerical value, refers to variations that can occur in the numerical value. For example, variations can occur through differences in the manufacture of a particular substrate or component. In one aspect, the term "about" means within 1%, 5%, or up to 10% of the recited numerical value.

As used herein, the polymer shell is a polymer surface of a bead. Due to the nature of the beads described herein, the contiguity particles can retain genetic material after multiple assays and can be released by physical force, cleaving chemicals, or by generating osmotic imbalance depending on the thickness of the polymer shell.

Hydrogels may be prepared by cross-linking hydrophilic biopolymers or synthetic polymers. Thus, in some aspects, the hydrogel may include a crosslinker. As used herein, the term "crosslinker" refers to a molecule that can form a three-dimensional network when reacted with the appropriate base monomers. Examples of the hydrogel polymers, which may include one or more crosslinkers, include but are not limited to, hyaluronans, chitosans, agar, heparin, sulfate, cellulose, alginates (including alginate sulfate), collagen, dextrans (including dextran sulfate), pectin, carrageenan, polylysine, gelatins (including gelatin type A), agarose, (meth)acrylate-oligolactide-PEO-oligolactide-(meth)acrylate, PEO-PPO-PEO copolymers (Pluronics), poly(phosphazene), poly(methacrylates), poly(N-vinylpyrrolidone), PL(G)A-PEO-PL(G)A copolymers, poly(ethylene imine), polyethylene glycol (PEG)-thiol, PEG-acrylate, acrylamide, N,N'-bis(acryloyl)cystamine, PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations thereof. Thus, for example, a combination may include a polymer and a crosslinker, for example polyethylene glycol (PEG)-thiol/PEG-acrylate, acrylamide/N,N'-bis(acryloyl)cystamine (BACy), or PEG/polypropylene oxide (PPO). In some aspects, the polymer shell includes a four-arm polyethylene glycol (PEG). In some aspects, the four-arm polyethylene glycol (PEG) is selected from the group consisting of PEG-acrylate, PEG-amine, PEG-carboxylate, PEG-dithiol, PEG-epoxide, PEG-isocyanate, and PEG-maleimide

In some aspect, the crosslinker is an instantaneous crosslinker or a slow crosslinker. An instantaneous crosslinker is a crosslinker that instantly crosslinks the hydrogel polymer, and is also referred to herein as click chemistry. Instantaneous crosslinkers may include dithiol oil + PEG-maleimide or PEG epoxide + amine oil. A slow crosslinker is a crosslinker that slowly crosslinks the hydrogel polymer, and may include PEG-epoxide + PEG-amine or PEG-dithiol + PEG-acrylate. A slow crosslinker may take more than several hours to crosslink, for example more than 2, 3, 4, 5, 6, 7, 8, 9, 10, 11, or 12 hours to crosslink. In some aspects provided herein, contiguity particles are formulated by an instantaneous crosslinker, and thereby preserve the state of the nucleic acid molecule better compared to a slow crosslinker.

In some aspects, a crosslinker forms a disulfide bond in the hydrogel polymer, thereby linking hydrogel polymers. In some aspects, the hydrogel polymers form a hydrogel matrix having pores (for example, a porous hydrogel matrix). These pores are capable of retaining sufficiently large particles, such as nucleic acids extracted therefrom within the polymer shell, but allow other materials, such as reagents, to pass through the pores, thereby passing in and out of the beads. In some aspects, the pore size of the polymer shell is finely tuned by varying the ratio of the concentration of polymer to the concentration of crosslinker. In some aspects, the ratio of polymer to crosslinker is 30:1, 25:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, or 1:30, or a ratio within a range defined by any two of the aforementioned ratios. In some aspects, additional functions such as DNA primer, or charged chemical groups can be grafted to polymer matrix to meet the requirements of different applications.

As used herein, the term "porosity" means the fractional volume (dimension-less) of a hydrogel that is composed of open space, for example, pores or other openings. Therefore, porosity measures void spaces in a material and is a fraction of volume of voids over the total volume, as a percentage between 0 and 100% (or between 0 and 1). Porosity of the hydrogel may range from 0.5 to 0.99, from about 0.75 to about 0.99, or from about 0.8 to about 0.95.

The polymer shell can have any pore size that allows for sufficient diffusion of reagents while concomitantly retaining the nucleic acids. As used herein, the term "pore size" refers to a diameter or an effective diameter of a cross-section of the pores. The term "pore size" can also refer to an average diameter or an average effective diameter of a cross-section of the pores, based on the measurements of a plurality of pores. The effective diameter of a cross-section that is not circular equals the diameter of a circular cross-section that has the same cross-sectional area as that of the non-circular cross-section. In some aspects, the hydrogel can be swollen when the hydrogel is hydrated. The sizes of the pores size can then change depending on the water content in the hydrogel. In some aspects, the pores of the hydrogel can have a pore of sufficient size to retain integrity of the hydrogel but allow reagents to pass through. In some aspects, the interior of the polymer shell is an aqueous environment. In some aspects, the nucleic acid molecule associated with the polymer shell is free from interaction with the polymer shell and/or is not in contact with the polymer shell.

In some aspects, the contiguity particle has a diameter of about 20 µm to about 200 µm, such as 20, 30, 40, 50, 60, 70, 80, 90, 100, 110, 120, 130, 140, 150, 160, 170, 180, 190, or 200 µm, or a diameter within a range defined by any two of the aforementioned values. The size of the contiguity particle may change due to environmental factors. In some aspects, the contiguity particles expand when they are separated from continuous oil phase and immersed in an aqueous phase. In some aspects, expansion of the contiguity particles increases the efficiency of performing assays on the genetic material. In some aspects, expansion of the contiguity particles creates a larger environment for indexed inserts to be amplified during PCR which may otherwise be restricted in current cell based assays.

In some aspects, pore size allows extracted nucleic acids to diffuse through the polymer shell. In some aspects, the pore size of the contiguity particles can be controlled by altering the crosslinking chemistry. The final crosslinked pore size can further be altered by changing the environment of the contiguity particle, for example, by changing salt concentration, pH, or temperature, thereby allowing immobilized molecules to be released from the contiguity particle.

In some aspects, the crosslinker is a reversible crosslinker. In some aspects, a reversible crosslinker is capable of reversibly crosslinking the hydrogel polymer and is capable of being un-crosslinked in the presence of a cleaver. In some aspects, a crosslinker can be cleaved by the presence of a reducing agent, by elevated temperature, or by an electric field. In some aspects, the reversible crosslinker may be N,N'-bis(acryloyl)cystamine, a reversible crosslinker for polyacrylamide gels, wherein a disulfide linkage may be broken in the presence of a suitable reducing agent. Bead porosity may be increased by temperature or chemical means, thereby releasing contacting the crosslinker with a reducing agent cleaves the disulfide bonds of the crosslinker, breaking down the beads. The beads degrade, and release the contents, such as nucleic acids that were retained therein. In some aspects, the crosslinker is cleaved by increasing the temperature to greater than 50, 55, 60, 65, 70, 75, 80, 85, 90, 95, or 100°C. In some aspects, the crosslinker is cleaved by contacting the beads with a reducing agent. In some aspects, the reducing agents include phosphine compounds, water soluble phosphines, nitrogen containing phosphines and salts and derivatives thereof, dithioerythritol (DTE), dithiothreitol (DTT) (cis and trans isomers, respectively, of 2,3-dihydroxy-1,4-dithiolbutane), 2-mercaptoethanol or β-mercaptoethanol (BME), 2-mercaptoethanol or aminoethanethiol, glutathione, thioglycolate or thioglycolic acid, 2,3-dimercaptopropanol, tris(2-carboxyethyl)phosphine (TCEP), tris(hydroxymethyl)phosphine (THP), or P-[tris(hydroxymethyl)phosphine] propionic acid (THPP).

In some aspects, elevating the temperature to increase diffusion or contacting with a reducing agent degrades the crosslinker.

In some aspects, the crosslinking of the crosslinker establishes pores within the contiguity particle. In some aspects, the size of the pores in the polymer shell are regulatable and are formulated to associate with transposome. The on bead transposome is formulated to associate with nucleic acids of greater than about 300 base pairs. In some aspects, the on bead transposome and nucleic acids may be subjected to various reagents for performance of various reactions. In some aspects, the reagents including reagents for processing genetic material, such as reagents for isolating nucleic acids from a cell, for amplifying or sequencing nucleic acids, or for preparation of nucleic acid libraries. In some aspects, reagents include, for example, lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

### Methods of Making Contiguity Particles

Some aspects provided herein relate to methods of making contiguity particles having transposome associated therewith. In some aspects, a contiguity particle is prepared by static means, such as by microwell/microarray methods or microdissection methods, without the need of a microfluidic device. Thus, in some aspects, the contiguity particles described herein are prepared by a device-free method. Initiation of polymerization can occur by chemical reaction of active group on monomer units with specific moieties of membrane proteins, glycans or other small molecules. Initial step of monomer polymerization can be followed by one or several rounds of monomer units deposition promoted by either electrostatic or hydrophobic forces. Some of monomer layers can contain functional groups such as biotin or other ligands that can be used later for specific association with transposome.

In some aspects, a contiguity particle is prepared by dynamic means, such as by vortex assisted emulsion, microfluidic droplet generation, or valve based microfluidics. As used herein, vortex assisted emulsion refers to vortexing a hydrogel polymer with a transposome in a container, such as in a tube, vial, or reaction vessel. The components can be mixed, for example by manual or mechanical vortexing or shaking. In some aspects, manual mixing results in beads that associate with transposome, wherein the beads have a size of 20, 25, 30, 35, 40, 45, 50, 55, 60, 65, 70, 75, 80, 85, 90, 95, 100, 110, 120, 130, 140, 150, 160, 170, 180, 190, or 200 µm in diameter, or a size within a range defined by any two of the aforementioned values. In some aspects, the size of the beads is non-uniform, and thus, the size of the beads includes beads of various diameters.

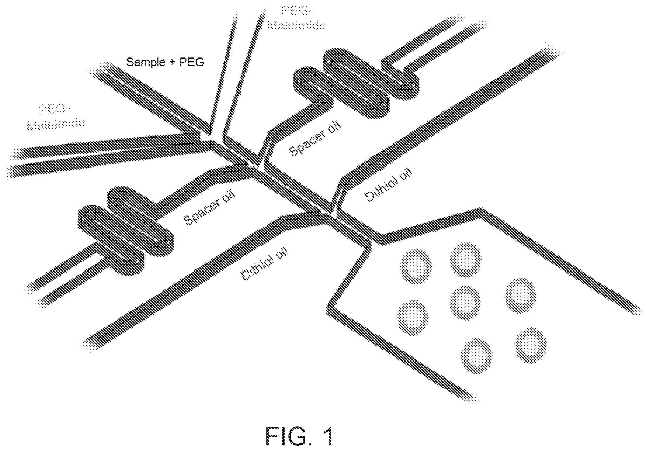

In some aspects, the contiguity particles are prepared by microfluidic flow techniques. Microfluidic flow includes use of a microfluidic device for assisted gel emulsion generation, as shown in FIG. 1. In some aspects, the microfluidic device includes microchannels configured to produce a contiguity particle of a desired size and configured to associate with transposome at a desired density. In some aspects, the microfluidic device has a height of 50, 60, 70, 80, 90, 100, 110, 120, 130, 140, 150, 160, 170, 180, 190, or 200 µm, or a height within a range defined by any two of the aforementioned values. In some aspects, the microfluidic device includes one or more channels. In some aspects, the microfluidic device includes a channel for introducing reagents to be associated with the contiguity particle, such as transposome, nucleic acids, or the like, that has been introduced to a polymer, a channel for introducing a crosslinker, and a channel for an immiscible fluid. In some embodiments, the width of the one or more channels is identical. In some aspects, the width of the one or more channels is different. In some aspects, the width of the one or more channels is 20, 30, 40, 45, 50, 55, 60, 65, 70, 75, 80, 85, 90, 95, 100, 105, 110, 115, 120, 125, 130, 135, 140, 145, or 150 µm, or a width within a range defined by any two of the aforementioned values. The width and height of the channel is not necessarily restricted to the values described herein and a person of skill in the art will recognize that the size of the contiguity particle will be dependent in part on the size of the channels of the microfluidic device. Thus, the size of the contiguity particle may be tuned in part by modifying the size of the channels. In addition to the size of the microfluidic device and the width of the channels, the flow rate of the channels may also affect the size of the contiguity particles, and may also effect the density of transposomes associated with each contiguity particle.

In some aspects, the flow rate of the transposome in the polymer through the microfluidic channel is 1, 2, 5, 10, 20, 30, 40, 50, 60, 70, 80, 90, 100, 110, 120, 130, 140, or 150 µL/min, or a rate within a range defined by any two of the aforementioned values. In some aspects, the flow rate of the crosslinker in the microfluidic channel is 1, 2, 5, 10, 20, 30, 40, 50, 60, 70, 80, 90, 100, 110, 120, 130, 140, or 150 µL/min, or a rate within a range defined by any two of the aforementioned values. In some aspects, the flow rate of the immiscible fluid in the microfluidic channel is 20, 30, 50, 80, 100, 150, 160, 170, 180, 190, 200, 225, 250, 275, 300, 325, 350, 375, or 400 µL/min, or a rate within a range defined by any two of the aforementioned values. In some aspects, the transposome mixed with the polymer and the crosslinker contact one another in the microfluidic droplet generator upstream of the immiscible fluid. The contiguity particles begin to form upon contact with the crosslinker, and associate with transposome. The forming contiguity particles continue to flow through the microfluidic droplet generator into an immiscible fluid, such as a spacer oil and/or a crosslinking oil, at a flow rate less than the flow rate of the immiscible fluid, thereby forming droplets. In some aspects, the immiscible fluid is introduced in two stages, as shown in FIG. 1, including as a spacer oil and as a crosslinker oil. In some aspects, the spacer oil is a mineral oil, a hydrocarbon oil, a silicon oil, a fluorocarbon oil, or a polydimethylsiloxane oil, or mixtures thereof. The spacer oil as used herein is used to avoid crosslinking of the polymer at the channel aqueous-oil interphase.

In some aspects, the contiguity particles are formed instantaneously by crosslinking with an instantaneous crosslinker. For example, transposome may be associated with the bead with a polymers like four-arm PEG maleimide or epoxide using a microfluidic droplet generator can be instantaneously crosslinked using crosslinkers that are dissolvable in oils such as mineral oil or fluorocarbon oils like HFE-7500, forming a crosslinking oil. In some aspects, the crosslinking oil includes toluene, acetone, tetrahydrofuran with dithiol, amine functional groups as in the case of toluene 3, 4 dithiol, 2, 4 diaminotoluene, hexane dithiol, which readily diffuse into the forming droplets thereby instantaneously crosslinking the contiguity particles.

In some aspects, the contiguity particles are formulated in a uniform size distribution. In some aspects, the size of the contiguity particles is finely tuned by adjusting the size of the microfluidic device, the size of the one or more channels, or the flow rate through the microfluidic channels. In some aspects, the resulting contiguity particle has a diameter ranging from 20 to 200 µm, for example, 20, 25, 30, 35, 40, 45, 50, 55, 60, 65, 70, 75, 80, 85, 90, 95, 100, 110, 120, 130, 140, 150, 160, 170, 180, 190, or 200 µm, or a diameter within a range defined by any two of the aforementioned values.

In some aspectss, the size and uniformity of the contiguity particles can be further controlled by contacting a hydrogel polymer prior to particle formation with a fluidic modifier, such as with an alcohol, including isopropyl alcohol. In the absence of isopropyl alcohol, contiguity particles form at a greater diameter than contiguity particles formed in the presence of isopropyl alcohol. Isopropyl alcohol influences the fluidic property of the hydrogel polymer, allowing modulation of the size of contiguity particles.

As will be recognized by those of skill in the art, the microfluidic device depicted in FIG. 1 is exemplary of a three channel microfluidic device, but the microfluidic device may be modified, varied, or altered to generate contiguity particles of a particular size or to generate contiguity particles formed from varied hydrogel materials or crosslinkers.

In some aspects, a contiguity particle, whether prepared by vortex assisted emulsion or microfluidic inertial flow assisted emulsion. In some aspects, the density of transposome associated with contiguity particle can be controlled by diluting or concentration the solution containing the transposome within the inputted sample. The sample including the transposome is mixed with hydrogel polymer, and the hydrogel polymer containing the transposome is submitted to vortex assisted emulsion or microfluidic flow assisted emulsion, as described herein.

In some aspects, the contiguity particles are functionalized with a nucleotide. In some aspects, the nucleotide is an oligonucleotide or polyT nucleotide. In some aspects, the nucleotide is bound to the contiguity particles, and the functionalized contiguity particles can be used for targeted capture of a nucleotide of interest.

In some aspects, the contiguity particles associated with a transposome are cured to sustain performing multiple co-assays on a single contiguity particle, including multiple buffer washes, multiple reagent exchanges, and multiple analyses based on the assay being performed. The formulated contiguity particles, prepared by any of the methods described herein, including surface-initiated polymerization techniques, vortexing, or by the microfluidic techniques may be loaded or seeded onto a patterned flow cell, a microarray, a plate with wells, an etched surface, a microfluidic channel, a bead, a column, or other surface for performing multiple co-assays.

### Methods of Linked Long Read Indexing

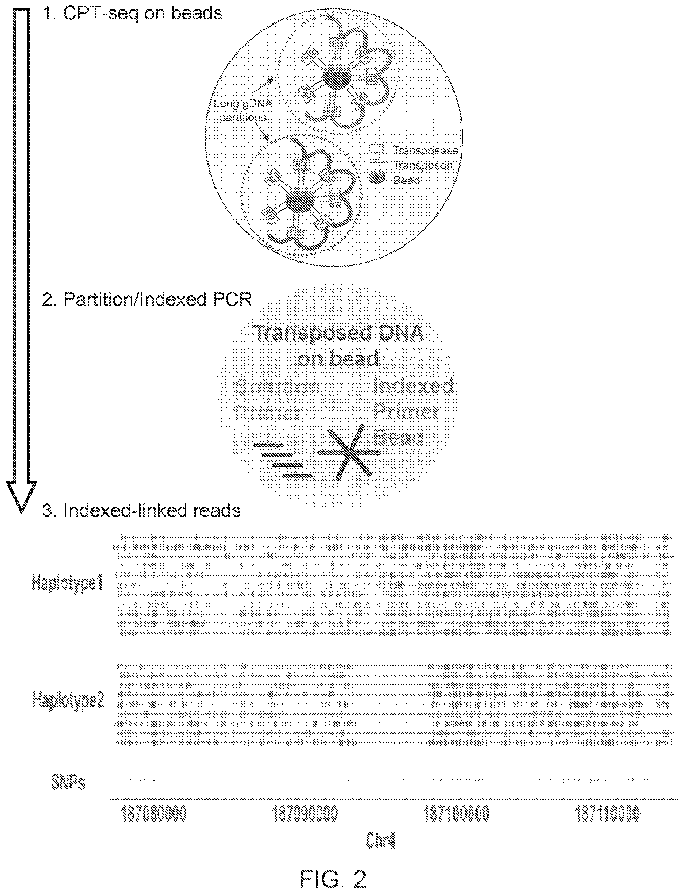

Some aspects provided herein relate to methods of performing linked long read indexing using the on bead tagmented contiguity particles. In some aspects, the method includes obtaining a contiguity particle as described herein, associating the particles with transposomes and nucleic acid molecules, performing on bead tagmentation, performing partition of the on tagmented beads, and indexed PCR. In some aspects, the methods include the steps as outlined in FIG. 2, which depicts a schematic representation for exemplary methods for performing linked long read indexing, including contiguity-preserving transposition sequencing (CPT-seq) on beads (step 1), partition/indexed PCR (step 2), and indexed-linked reads (step 3). The bead of step 1 is associated with a transposome, which includes a transposon and a transposase. A nucleic acid molecule is associated with the transposase, and undergoes tagmentation, for example CPT-seq. Following tagmentation, the on bead tagmentation products are partitioned using a droplet generator or other method. Such partitioned samples of transposed DNA on beads may be subjected to solution primers and indexed primer beads, which index the transposed DNA. In some aspects, the indexed DNA is subjected to indexed PCR and indexed-linked reads are obtained.

In addition to the contiguity particles (also referred to herein as contiguity beads), primer beads were also prepared. The primer beads can include hydrogel beads of materials, compositions, and formulations described herein with respect to the contiguity particles, but instead of being associated with transposome, include a primer (such as a P5 primer), a barcode, and an adapter (such as a Nextera adapter). A single primer bead may be partitioned together with a single contiguity particle within a partitioned droplet, and together with a primer solution mix, which may include adapters (such as B15 adapters) and primers (such as P7 primers). The partitioned droplets may be used for barcoded indexing, with a bead pool of more than 900,000 indexed barcodes.

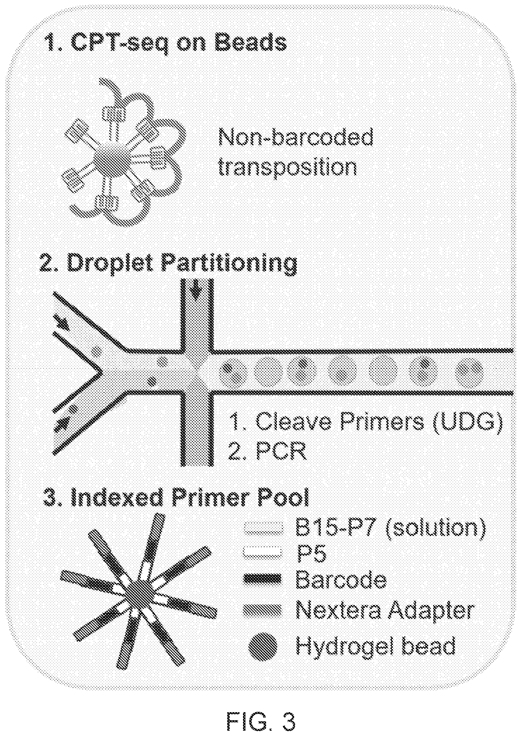

In some aspects, the methods includes the steps as outlined in FIG. 3, which depicts a schematic representation for exemplary methods for performing long read indexing, including CPT-seq on beads, droplet partitioning, and indexed primer pool indexing, using non-barcoded transposition.

In some aspects, the contiguity particles are prepared as described herein, and droplets are partitioned onto a surface, such as a flow cell device, a well of a plate, a slide, or a patterned surface. In some aspects, the surface is a flow cell device, and includes an insert having microwells or micropillars in an array for distribution of the contiguity particles for spatial indexing in a flow cell device. In some aspects, the droplets are partitioned onto a welled plate with a single contiguity particle in each well. A welled plate may include, for example, a 12 well plate, a 24 well plate, a 48 well plate, a 96 well plate, a 384 well plate, a 1536 well plate, a 3456 well plate, or a 9600 well plate, or any number of wells in a plate, with a single contiguity particle, and partitioning the contiguity particle into droplet indexing for nucleic acid indexing. In some aspects, the contiguity particles are subjected to multiple co-assays in sequence, including, for example, buffer washes, lysis, DNA analysis, RNA analysis, protein analysis, tagmentation, nucleic acid amplification, nucleic acid sequencing, DNA library preparation, assay for transposase accessible chromatic using sequencing (ATAC-seq), contiguity-preserving transposition sequencing (CPT-seq), or any combination thereof performed sequentially.

In some aspects, the contiguity particle associated with a transposome is treated to associate nucleic acids of interest thereto. In some aspects, nucleic acids of interest are isolated from a cell. For example, the cell may be contacted with a lysis buffer. As used herein, "lysis" means perturbation or alteration to a cell wall or viral particle facilitating access to or release of the cellular RNA or DNA. Neither complete disruption nor breakage of the cell wall is an essential requirement for lysis. By the term "lysis buffer" is meant a buffer that contains at least one lysing agent. Typical enzymatic lysing agents include, but are not limited to, lysozyme, glucolase, zymolose, lyticase, proteinase K, proteinase E, and viral endolysins and exolysins. Thus, for example, lysis of cells may be performed by introducing lysing agents, such as lysozyme and proteinase K. The gDNA from the cells is then associated with the contiguity particles. In some aspects, following lysis treatment, isolated nucleic acid is retained upon the contiguity particle, and may be used for further processing.

DNA analysis refers to any technique used to amplify, sequence, or otherwise analyze DNA. DNA amplification can be accomplished using PCR techniques or pyrosequencing. DNA analysis may also comprise non-targeted, non-PCR based DNA sequencing (e.g., metagenomics) techniques. As a non-limiting example, DNA analysis may include sequencing the hyper-variable region of the 16S rDNA (ribosomal DNA) and using the sequencing for species identification via DNA.

RNA analysis refers to any technique used to amplify, sequence, or otherwise analyze RNA. The same techniques used to analyze DNA can be used to amplify and sequence RNA. RNA, which is less stable than DNA is the translation of DNA in response to a stimuli. Therefore, RNA analysis may provide a more accurate picture of the metabolically active members of the community and may be used to provide information about the community function of organisms in a sample. Nucleic acid sequencing refers to use of sequencing to determine the order of nucleotides in a sequence of a nucleic acid molecule, such as DNA or RNA.

The term "sequencing," as used herein, refers to a method by which the identity of at least 10 consecutive nucleotides (e.g., the identity of at least 20, at least 50, at least 100 or at least 200 or more consecutive nucleotides) of a polynucleotide is obtained.

The terms "next-generation sequencing" or "high-throughput sequencing" or "NGS" generally refers to high throughput sequencing technologies, including, but not limited to, massively parallel signature sequencing, high throughput sequencing, sequencing by ligation (e.g., SOLiD sequencing), proton ion semiconductor sequencing, DNA nanoball sequencing, single molecule sequencing, and nanopore sequencing and may refer to the parallelized sequencing-by-synthesis or sequencing-by-ligation platforms currently employed by Illumina, Life Technologies, or Roche, etc. Next-generation sequencing methods may also include nanopore sequencing methods or electronic-detection based methods such as Ion Torrent technology commercialized by Life Technologies or single molecule fluorescence-based method commercialized by Pacific Biosciences.

Protein analysis refers to the study of proteins, and may include proteomic analysis, determination of post-translational modification of proteins of interest, determination of protein expression levels, or determination of protein interactions with other molecules, including with other proteins or with nucleic acids.

As used herein, the term "tagmentation" refers to the modification of DNA by a transposome complex comprising transposase enzyme complexed with adaptors comprising transposon end sequence. Tagmentation results in the simultaneous fragmentation of the DNA and ligation of the adaptors to the 5' ends of both strands of duplex fragments. Following a purification step to remove the transposase enzyme, additional sequences can be added to the ends of the adapted fragments, for example by PCR ligation, or any other suitable methodology known to those of skill in the art.

The method of the invention can use any transposase that can accept a transposase end sequence and fragment a target nucleic acid, attaching a transferred end, but not a non-transferred end. A "transposome" is comprised of at least a transposase enzyme and a transposase recognition site. In some such systems, termed "transposomes", the transposase can form a functional complex with a transposon recognition site that is capable of catalyzing a transposition reaction. The transposase or integrase may bind to the transposase recognition site and insert the transposase recognition site into a target nucleic acid in a process sometimes termed "tagmentation". In some such insertion events, one strand of the transposase recognition site may be transferred into the target nucleic acid.

In standard sample preparation methods, each template contains an adaptor at either end of the insert and often a number of steps are required to both modify the DNA or RNA and to purify the desired products of the modification reactions. These steps are performed in solution prior to the addition of the adapted fragments to a flow cell where they are coupled to the surface by a primer extension reaction that copies the hybridized fragment onto the end of a primer covalently attached to the surface. These 'seeding' templates then give rise to monoclonal clusters of copied templates through several cycles of amplification.

The number of steps required to transform DNA into adaptor-modified templates in solution ready for cluster formation and sequencing can be minimized by the use of transposase mediated fragmentation and tagging.

In some aspects, transposon based technology can be utilized for fragmenting DNA, for example as exemplified in the workflow for Nextera^{™} DNA sample preparation kits (Illumina, Inc.) wherein genomic DNA can be fragmented by an engineered transposome that simultaneously fragments and tags input DNA ("tagmentation") thereby creating a population of fragmented nucleic acid molecules which comprise unique adapter sequences at the ends of the fragments.

Some aspects can include the use of a hyperactive Tn5 transposase and a Tn5-type transposase recognition site (Gory shin and Reznikoff, J. Biol. Chem., 273:7367 (1998)), or MuA transposase and a Mu transposase recognition site comprising R1 and R2 end sequences (Mizuuchi, K., Cell, 35: 785, 1983; Savilahti, H, et al., EMBO J., 14: 4893, 1995). An exemplary transposase recognition site that forms a complex with a hyperactive Tn5 transposase (e.g., EZ-Tn5^{™} Transposase, Epicentre Biotechnologies, Madison, Wis.).

More examples of transposition systems that can be used with certain aspects provided herein include Staphylococcus aureus Tn552 (Colegio et al., J. Bacteriol., 183: 2384-8, 2001; Kirby C et al., Mol. Microbiol., 43: 173-86, 2002), Ty1 (Devine & Boeke, Nucleic Acids Res., 22: 3765-72, 1994 and International Publication WO 95/23875), Transposon Tn7 (Craig, N L, Science. 271: 1512, 1996; Craig, N L, Review in: Curr Top Microbiol Immunol., 204:27-48, 1996), Tn/O and IS10 (Kleckner N, et al., Curr Top Microbiol Immunol., 204:49-82, 1996), Mariner transposase (Lampe D J, et al., EMBO J., 15: 5470-9, 1996), Tc1 (Plasterk RH, Curr. Topics Microbiol. Immunol., 204: 125-43, 1996), P Element (Gloor, GB, Methods Mol. Biol., 260: 97-114, 2004), Tn3 (Ichikawa & Ohtsubo, J Biol. Chem. 265:18829-32, 1990), bacterial insertion sequences (Ohtsubo & Sekine, Curr. Top. Microbiol. Immunol. 204: 1-26, 1996), retroviruses (Brown, et al., Proc Natl Acad Sci USA, 86:2525-9, 1989), and retrotransposon of yeast (Boeke & Corces, Annu Rev Microbiol. 43:403-34, 1989). More examples include IS5, Tn10, Tn903, IS911, and engineered versions of transposase family enzymes (Zhang et al., (2009) PLoS Genet. 5:e1000689. Epub 2009 Oct. 16; Wilson C. et al (2007) J. Microbiol. Methods 71:332-5).

Assay for transposase accessible chromatic using sequencing (ATAC-seq) refers to a rapid and sensitive method of integrative epigenomic analysis. ATAC-seq captures open chromatin sites and reveals interplay between genomic locations of open chromatin, DNA binding proteins, individual nucleosomes, and higher-order compaction at regulatory regions with nucleotide resolution. Classes of DNA binding factor that strictly avoid, can tolerate, or tend to overlap with nucleosomes have been discovered. Using ATAC-seq, the serial daily epigenomes of resting human T cells was measured and evaluated from a pro band via standard blood draws, demonstrating the feasibility of reading personal epigenomes in clinical timescales for monitoring health and disease. More specifically, ATAC-seq may be performed by treating chromatin from a cell with an insertional enzyme complex to produce tagged fragments of genomic DNA. In this step, the chromatin is tagmented (for example, fragmented and tagged in the same reaction) using an insertional enzyme such as Tn5 or MuA that cleaves the genomic DNA in open regions in the chromatin and adds adaptors to both ends of the fragments.

In some cases, the conditions may be adjusted to obtain a desirable level of insertion in the chromatin (e.g., an insertion that occurs, on average, every 50 to 200 base pairs in open regions). The chromatin used in the method may be made by any suitable method. In some aspects, nuclei may be isolated, lysed, and the chromatin may be further purified, e.g., from the nuclear envelope. In other aspects, the chromatin may be isolated by contacting isolated nuclei with the reaction buffer. In these aspects, the isolated nuclei may lyse when it makes contact with the reaction buffer (which comprises insertional enzyme complexes and other necessary reagents), which allows the insertional enzyme complexes access to the chromatin. In these aspects, the method may comprise isolating nuclei from a population of cells; and combining the isolated nuclei with the transposase and adaptors, wherein the combining results in both lysis of the nuclei to release said chromatin and production of the adaptor-tagged fragments of genomic DNA. The chromatin does not require cross-linking as in other methods (e.g., ChIP-SEQ methods).

After the chromatin has been fragmented and tagged to produce tagged fragments of genomic DNA, at least some of the adaptor tagged fragments are sequenced to produce a plurality of sequence reads. The fragments may be sequenced using any suitable method. For example, the fragments may be sequenced using Illumina's reversible terminator method, Roche's pyrosequencing method (454), Life Technologies' sequencing by ligation (the SOLiD platform) or Life Technologies' Ion Torrent platform. Examples of such methods are described in the following references: Margulies et al. (Nature 2005 437: 376-80); Ronaghi et al. (Analytical Biochemistry 1996 242: 84-9); Shendure et al. (Science 2005 309: 1728-32); Imelfort et al. (Brief Bioinform. 2009 10:609-18); Fox et al. (Methods Mol Biol. 2009;553:79-108); Appleby et al. (Methods Mol Biol. 2009; 513:19-39) and Morozova et al. (Genomics. 2008 92:255-64). As would be apparent, forward and reverse sequencing primer sites that are compatible with a selected next generation sequencing platform can be added to the ends of the fragments during the amplification step. In certain aspects, the fragments may be amplified using PCR primers that hybridize to the tags that have been added to the fragments, where the primer used for PCR have 5' tails that are compatible with a particular sequencing platform. Methods of performing ATAC-seq are set forth in PCT Application No. PCT/US2014/038825.

The term "chromatin," as used herein, refers to a complex of molecules including proteins and polynucleotides (e.g. DNA, RNA), as found in a nucleus of a eukaryotic cell. Chromatin is composed in part of histone proteins that form nucleosomes, genomic DNA, and other DNA binding proteins (e.g., transcription factors) that are generally bound to the genomic DNA.

Contiguity-preserving transposition sequencing (CPT-seq) refers to a method of sequencing while preserving contiguity information by the use of transposase to maintain the association of template nucleic acid fragments adjacent in the target nucleic acid. For example, CPT may be carried out on a nucleic acid, such as on DNA or RNA. The CPT-nucleic acid can be captured by hybridization of complimentary oligonucleotides having unique indexes or barcodes and immobilized on a solid support. In some aspects, the oligonucleotide immobilized on the solid support may further comprise primer binding sites, unique molecular indices, in addition to barcodes. Advantageously, such use of transposomes to maintain physical proximity of fragmented nucleic acids increases the likelihood that fragmented nucleic acids from the same original molecule, e.g., chromosome, will receive the same unique barcode and index information from the oligonucleotides immobilized on a solid support. This will result in a contiguously-linked sequencing library with unique barcodes. The contiguously-linked sequencing library can be sequenced to derive contiguous sequence information. The contiguity particles described herein may be contacted with the CPT-seq reagents for performance of CPT-seq on nucleic acids extracted from a cell.

As used herein the term "contiguity information" refers to a spatial relationship between two or more DNA fragments based on shared information. The shared aspect of the information can be with respect to adjacent, compartmental and distance spatial relationships. Information regarding these relationships in turn facilitates hierarchical assembly or mapping of sequence reads derived from the DNA fragments. This contiguity information improves the efficiency and accuracy of such assembly or mapping because traditional assembly or mapping methods used in association with conventional shotgun sequencing do not take into account the relative genomic origins or coordinates of the individual sequence reads as they relate to the spatial relationship between the two or more DNA fragments from which the individual sequence reads were derived.

Therefore, according to the aspects described herein, methods of capturing contiguity information may be accomplished by short range contiguity methods to determine adjacent spatial relationships, mid-range contiguity methods to determine compartmental spatial relationships, or long range contiguity methods to determine distance spatial relationships. These methods facilitate the accuracy and quality of DNA sequence assembly or mapping, and may be used with any sequencing method, such as those described herein.

Contiguity information includes the relative genomic origins or coordinates of the individual sequence reads as they relate to the spatial relationship between the two or more DNA fragments from which the individual sequence reads were derived. In some aspects, contiguity information includes sequence information from non-overlapping sequence reads.

In some aspects, the contiguity information of a target nucleic acid sequence is indicative of haplotype information. In some aspects, the contiguity information of a target nucleic acid sequence is indicative of genomic variants.

Single cell combinatorial indexed sequencing (SCI-seq) is a sequencing technique for simultaneously generating thousands of low-pass single cell libraries for somatic copy number variant detection.

Accordingly, multiple co-assays may be performed on contiguity particles for purposes of analyzing nucleic acids, including assays described herein, alone or in combination with any other assay.

The indexed contiguity particles can also be loaded directly onto the flow cells held through an array of posts/microwells. The indexed libraries released from the contiguity particles (chemical/temperature release) and bind to the flow cell. This allows a powered indexing approach where the first level of indexing comes from spatial location and then the next level comes from the indexed libraries from a single contiguity particle. Alternatively, indexed libraries extracted from the contiguity particles can be collectively loaded onto the flow cell.

In some aspects, a contiguity particle is contacted with one or more reagents for nucleic acid processing. In some aspects, reagents can include lysis agents, nucleic acid purification agents, DNA amplification agents, tagmentation agents, PCR agents, or other agents used in processing of genetic materials. Thus, the contiguity particle provides a microenvironment for controlled reactions of nucleic acids.

In some aspects, entire DNA library preparation can be accomplished seamlessly inside the contiguity particle with multiple reagent exchanges by passing through the porous hydrogel while retaining the gDNA and its library products within the polymer shell. The hydrogel may be resistant to high temperatures up to 95°C for several hours to support different biochemical reactions.

As used herein, the terms "isolated," "to isolate," "isolation," "purified," "to purify," "purification," and grammatical equivalents thereof as used herein, unless specified otherwise, refer to the reduction in the amount of at least one contaminant (such as protein and/or nucleic acid sequence) from a sample or from a source (e.g., a cell) from which the material is isolated. Thus purification results in an "enrichment," for example, an increase in the amount of a desirable protein and/or nucleic acid sequence in the sample.

Following lysis and isolation of nucleic acids, amplification may be performed, such as multiple displacement amplification (MDA), which is a widely used technique for amplifying low quantities of DNA, especially from single cells. In some aspects, the nucleic acids are amplified, sequenced, or used for the preparation of nucleic acid libraries. As used herein, the terms "amplify" or "amplified" "amplifying" as used in reference to a nucleic acid or nucleic acid reactions, refer to in vitro methods of making copies of a particular nucleic acid, such as a target nucleic acid, or a nucleic acid associated with a contiguity particle, for example, by an aspects of the present disclosure. Numerous methods of amplifying nucleic acids are known in the art, and amplification reactions include polymerase chain reactions, ligase chain reactions, strand displacement amplification reactions, rolling circle amplification reactions, multiple annealing and looping based amplification cycles (MALBAC), transcription-mediated amplification methods such as NASBA, loop mediated amplification methods (e.g., "LAMP" amplification using loop-forming sequences. The nucleic acid that is amplified can be DNA comprising, consisting of, or derived from DNA or RNA or a mixture of DNA and RNA, including modified DNA and/or RNA. The products resulting from amplification of a nucleic acid molecule or molecules (for example, "amplification products"), whether the starting nucleic acid is DNA, RNA or both, can be either DNA or RNA, or a mixture of both DNA and RNA nucleosides or nucleotides, or they can comprise modified DNA or RNA nucleosides or nucleotides. A "copy" does not necessarily mean perfect sequence complementarity or identity to the target sequence. For example, copies can include nucleotide analogs such as deoxyinosine or deoxyuridine, intentional sequence alterations (such as sequence alterations introduced through a primer comprising a sequence that is hybridizable, but not complementary, to the target sequence, and/or sequence errors that occur during amplification.

The nucleic acids that are associated with the contiguity particle can be amplified according to any suitable amplification methodology known in the art. In some aspects, the nucleic acids are amplified on the contiguity particle. In some aspects, the contiguity particle is captured on a solid support and degraded, wherein the nucleic acids are released onto the solid support, and the nucleic acids are amplified on the solid support.

It will be appreciated that any of the amplification methodologies described herein or generally known in the art can be utilized with universal or target-specific primers to amplify nucleic acids. Suitable methods for amplification include, but are not limited to, the polymerase chain reaction (PCR), strand displacement amplification (SDA), transcription mediated amplification (TMA) and nucleic acid sequence based amplification (NASBA), as described in U.S. Pat. No. 8,003,354. The above amplification methods can be employed to amplify one or more nucleic acids of interest. For example, PCR including multiplex PCR, SDA, TMA, NASBA and the like can be utilized to amplify nucleic acids. In some aspects, primers directed specifically to the nucleic acid of interest are included in the amplification reaction.

Other suitable methods for amplification of nucleic acids can include oligonucleotide extension and ligation, rolling circle amplification (RCA) (Lizardi et al., Nat. Genet. 19:225-232 (1998)) and oligonucleotide ligation assay (OLA) technologies (See generally U.S. Pat. Nos. 7,582,420, 5,185,243, 5,679,524 and 5,573,907; EP 0 320 308 B1; EP 0 336 731 B1; EP 0 439 182 B1; WO 90/01069; WO 89/12696; and WO 89/09835). It will be appreciated that these amplification methodologies can be designed to amplify nucleic acids. For example, in some aspects, the amplification method can include ligation probe amplification or oligonucleotide ligation assay (OLA) reactions that contain primers directed specifically to the nucleic acid of interest. In some aspects, the amplification method can include a primer extension-ligation reaction that contains primers directed specifically to the nucleic acid of interest, and which are capable of passing through the hydrogel pores. As a non-limiting example of primer extension and ligation primers that can be specifically designed to amplify a nucleic acid of interest, the amplification can include primers used for the GoldenGate assay (Illumina, Inc., San Diego, Calif.) as exemplified by U.S. Pat. Nos. 7,582,420 and 7,611,869. In each of the methods described, the reagents and components involved in the nucleic acid reaction are capable of passing through the pores of the contiguity particle while retaining the nucleic acid itself within the contiguity particle.

In some aspects, the nucleic acids are amplified using cluster amplification methodologies as exemplified by the disclosures of U.S. Pat. Nos. 7,985,565 and 7,115,400. The materials of U.S. Pat. Nos. 7,985,565 and 7,115,400 describe methods of nucleic acid amplification which allow amplification products to be immobilized on a solid support in order to form arrays comprised of clusters or "colonies" of immobilized nucleic acid molecules. Each cluster or colony on such an array is formed from a plurality of identical immobilized polynucleotide strands and a plurality of identical immobilized complementary polynucleotide strands. The arrays so-formed are generally referred to herein as "clustered arrays". The products of solid-phase amplification reactions such as those described in U.S. Pat. Nos. 7,985,565 and 7,115,400 are so-called "bridged" structures formed by annealing of pairs of immobilized polynucleotide strands and immobilized complementary strands, both strands being immobilized on the solid support at the 5' end, preferably via a covalent attachment. Cluster amplification methodologies are examples of methods wherein an immobilized nucleic acid template is used to produce immobilized amplicons. Other suitable methodologies can also be used to produce immobilized amplicons from immobilized DNA fragments produced according to the methods provided herein. For example one or more clusters or colonies can be formed via solid-phase PCR whether one or both primers of each pair of amplification primers are immobilized. In some aspects, the nucleic acids are amplified on the contiguity particle, and then deposited in an array or on a solid support in a cluster.

Additional amplification methods include isothermal amplification. Exemplary isothermal amplification methods that can be used include, but are not limited to, multiple displacement amplification (MDA) as exemplified by, for example Dean et al., Proc. Natl. Acad. Sci. USA 99:5261-66 (2002) or isothermal strand displacement nucleic acid amplification exemplified by, for example U.S. Pat. No. 6,214,587. Other non-PCR-based methods that can be used in the present disclosure include, for example, strand displacement amplification (SDA) which is described in, for example Walker et al., Molecular Methods for Virus Detection, Academic Press, Inc., 1995; U.S. Pat. Nos. 5,455,166, and 5,130,238, and Walker et al., Nucl. Acids Res. 20:1691-96 (1992) or hyperbranched strand displacement amplification which is described in, for example Lage et al., Genome Research 13:294-307 (2003). Isothermal amplification methods can be used with the strand-displacing Phi 29 polymerase or Bst DNA polymerase large fragment, 5'->3' exo-for random primer amplification of genomic DNA. The use of these polymerases takes advantage of their high processivity and strand displacing activity. High processivity allows the polymerases to produce fragments that are 10-20 kb in length. As set forth above, smaller fragments can be produced under isothermal conditions using polymerases having low processivity and strand-displacing activity such as Klenow polymerase. Additional description of amplification reactions, conditions and components are set forth in detail in the disclosure of U.S. Pat. No. 7,670,810. In some aspects, the polymerases, reagents, and components required to perform these amplification reactions are capable of passing through the pores of the contiguity particles to interact with the nucleic acids, thereby amplifying the nucleic acids within the contiguity particles. In some aspects, random hexamers are annealed to the denatured DNA followed by strand displacement synthesis at a constant temperature in the presence of a catalyzing enzyme, Phi 29. This results in DNA amplification within the contiguity particles as confirmed by an increase in the fluorescence intensity (DNA was stained with SYTOX) after MDA. Independently, Nextera based tagmentation after lysis and clean up and subsequent gDNA amplification via PCR as indicated by a substantial increase in fluorescence intensity within the contiguity particles after Nextera tagmentation and PCR may also be performed. After this Nextera library preparation, the contiguity particles may be heated to 80°C for 3 minutes to release the contents of the contiguity particles namely, the sequencing ready library products from a cell.

Another nucleic acid amplification method that is useful in the present disclosure is Tagged PCR which uses a population of two-domain primers having a constant 5' region followed by a random 3' region as described, for example, in Grothues et al. Nucleic Acids Res. 21(5): 1321-2 (1993). The first rounds of amplification are carried out to allow a multitude of initiations on heat denatured DNA based on individual hybridization from the randomly-synthesized 3' region. Due to the nature of the 3' region, the sites of initiation are contemplated to be random throughout the genome. Thereafter, the unbound primers can be removed and further replication can take place using primers complementary to the constant 5' region.

In some aspects, the nucleic acids are sequenced in full or in part on the contiguity particles. The nucleic acids can be sequenced according to any suitable sequencing methodology, such as direct sequencing, including sequencing by synthesis, sequencing by ligation, sequencing by hybridization, nanopore sequencing and the like.

One sequencing methodology is sequencing-by-synthesis (SBS). In SBS, extension of a nucleic acid primer along a nucleic acid template (e.g. a target nucleic acid or amplicon thereof) is monitored to determine the sequence of nucleotides in the template. The underlying chemical process can be polymerization (e.g. as catalyzed by a polymerase enzyme). In a particular polymerase-based SBS aspect, fluorescently labeled nucleotides are added to a primer (thereby extending the primer) in a template dependent fashion such that detection of the order and type of nucleotides added to the primer can be used to determine the sequence of the template.

One or more amplified nucleic acids can be subjected to an SBS or other detection technique that involves repeated delivery of reagents in cycles. For example, to initiate a first SBS cycle, one or more labeled nucleotides, DNA polymerase, etc., can be flowed into/through a contiguity particle that houses one or more amplified nucleic acid molecules. Those sites where primer extension causes a labeled nucleotide to be incorporated can be detected. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for aspects that use reversible termination, a deblocking reagent can be delivered to the flow cell (before or after detection occurs). Washes can be carried out between the various delivery steps. The cycle can then be repeated n times to extend the primer by n nucleotides, thereby detecting a sequence of length n. Exemplary SBS procedures, fluidic systems and detection platforms that can be readily adapted for use with amplicons produced by the methods of the present disclosure are described, for example, in Bentley et al., Nature 456:53-59 (2008), WO 04/018497; U.S. Pat. No. 7,057,026; WO 91/06678; WO 07/123744; U.S. Pat. No. 7,329,492; U.S. Pat. No. 7,211,414; U.S. Pat. No. 7,315,019; U.S. Pat. No. 7,405,281, and US 2008/0108082.

Other sequencing procedures that use cyclic reactions can be used, such as pyrosequencing. Pyrosequencing detects the release of inorganic pyrophosphate (PPi) as particular nucleotides are incorporated into a nascent nucleic acid strand (Ronaghi, et al., Analytical Biochemistry 242(1), 84-9 (1996); Ronaghi, Genome Res. 11(1), 3-11 (2001); Ronaghi et al. Science 281(5375), 363 (1998); U.S. Pat. No. 6,210,891; U.S. Pat. No. 6,258,568 and U.S. Pat. No. 6,274,320). In pyrosequencing, released PPi can be detected by being immediately converted to adenosine triphosphate (ATP) by ATP sulfurylase, and the level of ATP generated can be detected via luciferase-produced photons. Thus, the sequencing reaction can be monitored via a luminescence detection system. Excitation radiation sources used for fluorescence based detection systems are not necessary for pyrosequencing procedures. Useful fluidic systems, detectors and procedures that can be adapted for application of pyrosequencing to amplicons produced according to the present disclosure are described, for example, in WIPO Pat. App. Ser. No. PCT/US11/57111, US 2005/0191698 A1, U.S. Pat. No. 7,595,883, and U.S. Pat. No. 7,244,559.

Some aspects can utilize methods involving the real-time monitoring of DNA polymerase activity. For example, nucleotide incorporations can be detected through fluorescence resonance energy transfer (FRET) interactions between a fluorophore-bearing polymerase and γ-phosphate-labeled nucleotides, or with zero mode waveguides (ZMWs). Techniques and reagents for FRET-based sequencing are described, for example, in Levene et al. Science 299, 682-686 (2003); Lundquist et al. Opt. Lett. 33, 1026-1028 (2008); Korlach et al. Proc. Natl. Acad. Sci. USA 105, 1176-1181 (2008).

Some SBS aspects include detection of a proton released upon incorporation of a nucleotide into an extension product. For example, sequencing based on detection of released protons can use an electrical detector and associated techniques that are commercially available. Examples of such sequencing systems are pyrosequencing (e.g. commercially available platform from 454 Life Sciences a subsidiary of Roche), sequencing using γ-phosphate-labeled nucleotides (e.g. commercially available platform from Pacific Biosciences) and sequencing using proton detection (e.g. commercially available platform from Ion Torrent subsidiary of Life Technologies) or sequencing methods and systems described in US 2009/0026082 A1; US 2009/0127589 A1; US 2010/0137143 A1; or US 2010/0282617 A1. Methods set forth herein for amplifying target nucleic acids using kinetic exclusion can be readily applied to substrates used for detecting protons. More specifically, methods set forth herein can be used to produce clonal populations of amplicons that are used to detect protons.

Another sequencing technique is nanopore sequencing (see, for example, Deamer et al. Trends Biotechnol. 18, 147-151 (2000); Deamer et al. Acc. Chem. Res. 35:817-825 (2002); Li et al. Nat. Mater. 2:611-615 (2003)). In some nanopore aspects, the target nucleic acid or individual nucleotides removed from a target nucleic acid pass through a nanopore. As the nucleic acid or nucleotide passes through the nanopore, each nucleotide type can be identified by measuring fluctuations in the electrical conductance of the pore. (U.S. Pat. No. 7,001,792; Soni et al. Clin. Chem. 53, 1996-2001 (2007); Healy, Nanomed. 2, 459-481 (2007); Cockroft et al. J. Am. Chem. Soc. 130, 818-820 (2008)).

Exemplary methods for array-based expression and genotyping analysis that can be applied to detection according to the present disclosure are described in U.S. Pat. No. 7,582,420; 6,890,741; 6,913,884 or 6,355,431 or US Pat. Pub. Nos. 2005/0053980 A1; 2009/0186349 A1 or US 2005/0181440 A1.

In the methods of isolating nucleic acids, amplification, and sequencing as described herein, various reagents are used for nucleic acid isolation and preparation. Such reagents may include, for example, lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations. These reagents pass through the pores of the contiguity particles, whereas the genetic material is retained within the contiguity particles. An advantage of the methods set forth herein is that they provide for a microenvironment for the processing of nucleic acids on a contiguity particle.

Adaptors can include sequencing primer sites, amplification primer sites, and indexes. As used herein an "index" can include a sequence of nucleotides that can be used as a molecular identifier and/or barcode to tag a nucleic acid, and/or to identify the source of a nucleic acid. In some aspects, an index can be used to identify a single nucleic acid, or a subpopulation of nucleic acids. In some aspects, nucleic acid libraries can be prepared within a contiguity particle. In some aspects, a single cell may be processed to obtain nucleic acids to be associated with a contiguity particle, and then may be used for combinatorial indexing of the nucleic acids, for example, using a contiguity preserving transposition sequencing (CPT-seq) approach. In some aspects, DNA from a single cell may be barcoded by encapsulation of single cell after WGA amplification with barcoded transposons.

Aspects of the "spatial indexing" methods and techniques described herein shortens data analysis and simplifies the process of library preparation from single cells and long DNA molecules. Existing protocols for single cell sequencing requires efficient physical separation of the cells and uniquely barcoding each isolated cell and pooling everything back together to do sequencing. Current protocols for synthetic long reads also requires cumbersome barcoding steps, and pooling each barcoded fragments together for sequencing and letting data analysis to distinguish genetic information coming from each barcoded cell. During these long processes there is also loss of genetic material which causes dropouts in the sequences. Aspects described herein not only shorten the process but also increase data resolution for single cells. Furthermore, aspects provided herein simplify the assembly of genomes of new organisms. Aspects described herein may be used to reveal rare genetic variations and co-occurrence of mutations. In some aspects, DNA library confined in the contiguity particles until release provide the opportunity to control the size of the fragments that is released on the surface by controlling the release process and hydrogel formulation.

In some aspects, the library may be amplified using primer sites in the adaptor sequences, and sequenced using sequencing primer sites in the adaptor sequences. In some aspects the adaptor sequences can include indexes to identify the source of the nucleic acids. The efficiency of subsequent amplification steps can be reduced by the formation of primer-dimers. To increase the efficiency of subsequent amplification steps, non-ligated single-stranded adaptors can be removed from ligation products.

### Preparing Nucleic Acid Libraries with Contiguity Particles

Some aspects of the systems, methods and compositions provided herein include methods in which adaptors are ligated to target nucleic acids. Adaptors can include sequencing primer binding sites, amplification primer binding sites, and indexes. For example, an adaptor can include a P5 sequence, a P7 sequence, or a complement thereof. As used herein a P5 sequence comprises a sequence defined by SEQ ID NO: 1 (AATGATACGGCGACCACCGA) and a P7 sequence comprises a sequence defined by SEQ ID NO: 2 (CAAGCAGAAGACGGCATACGA). In some aspects, the P5 or P7 sequence can further include a spacer polynucleotide, which may be from 1 to 20, such as 1 to 15, or 1 to 10, nucleotides, such as 2, 3, 4, 5, 6, 7, 8, 9, or 10 nucleotides in length. In some aspects, the spacer includes 10 nucleotides. In some aspects, the spacer is a polyT spacer, such as a 10T spacer. Spacer nucleotides may be included at the 5' ends of polynucleotides, which may be attached to a suitable support via a linkage with the 5' end of the polynucleotide. Attachment can be achieved through a sulphur-containing nucleophile, such as phosphorothioate, present at the 5' end of the polynucleotide. In some aspects, the polynucleotide will include a polyT spacer and a 5' phosphorothioate group. Thus, in some aspects, the P5 sequence is 5'phosphorothioate-TTTTTTTTTTAATGATACGGCGACCACCGA-3' (SEQ ID NO: 3), and in some aspects, the P7 sequence is 5'phosphorothioate-TTTTTTTTTT CAAGCAGAAGACGGCATACGA-3' (SEQ ID NO: 4).

Indexes can be useful to identify the source of a nucleic acid molecule. In some aspects, an adaptor can be modified to prevent the formation of concatemers, for example by the addition of blocking groups that prevent extension of the adaptor at one or both ends. Examples of 3' blocking groups include a 3'-spacer C3, a dideoxynucleotide, and attachment to a substrate. Examples of 5' blocking groups include a dephosphorylated 5' nucleotide, and attachment to a substrate.

Adaptors include nucleic acids, such as single-stranded nucleic acids. Adaptors can include short nucleic acids having a length less than, greater than, or equal to about 5 nucleotides, 10 nucleotides, 20 nucleotides, 30 nucleotides, 40 nucleotides, 50 nucleotides, 60 nucleotides, 70 nucleotides, 80 nucleotides, 90 nucleotides, 100 nucleotides, or a range between any two of the foregoing sizes. In some aspects, the adaptors are of sufficient size to pass through the pores of the contiguity particles. Target nucleic acids include DNA, such as genomic or cDNA; RNA, such as mRNA, sRNA or rRNA; or a hybrid of DNA and RNA. The nucleic acid can be isolated from a single cell. A nucleic acid can contain phosphodiester bonds, and can include other types of backbones, comprising, for example, phosphoramide, phosphorothioate, phosphorodithioate, O-methylphosphoroamidite and peptide nucleic acid backbones and linkages. A nucleic acid can contain any combination of deoxyribo- and ribonucleotides, and any combination of bases, including uracil, adenine, thymine, cytosine, guanine, inosine, xanthanine, hypoxanthanine, isocytosine, isoguanine, and base analogs such as nitropyrrole (including 3-nitropyrrole) and nitroindole (including 5-nitroindole). In some aspects, a nucleic acid can include at least one promiscuous base. A promiscuous base can base-pair with more than one different type of base and can be useful, for example, when included in oligonucleotide primers or inserts that are used for random hybridization in complex nucleic acid samples such as genomic DNA samples. An example of a promiscuous base includes inosine that may pair with adenine, thymine, or cytosine. Other examples include hypoxanthine, 5-nitroindole, acylic 5-nitroindole, 4-nitropyrazole, 4-nitroimidazole and 3-nitropyrrole. Promiscuous bases that can base-pair with at least two, three, four or more types of bases can be used.

Target nucleic acids can include a sample in which the average size of a nucleic acid in the sample is less than, greater than, or equal to about 2 kb, 1 kb, 500 bp, 400 bp, 200 bp, 100 bp, 50 bp, or a range between any two of the foregoing sizes. In some aspects, the average size of a nucleic acid in the sample is less than, greater than, or equal to about 2000 nucleotides, 1000 nucleotides, 500 nucleotides, 400 nucleotides, 200 nucleotides, 100 nucleotides, 50 nucleotides, or a range between any two of the foregoing sizes. In some aspects, the nucleic acid is of sufficient size that the nucleic acid is entrapped within the contiguity particle such that it cannot pass through the pores of the contiguity particle.

An example method includes dephosphorylating the 5' ends of target nucleic acids to prevent the formation of concatemers in subsequent ligation steps; ligating first adaptors to the 3' ends of the dephosphorylated targets using a ligase, in which the 3' ends of the first adaptors are blocked; re-phosphorylating of the 5' ends of the ligated targets; ligating a second adaptor to the 5' ends of the dephosphorylated targets using the single-stranded ligase, in which the 5' ends of the second adaptors are non-phosphorylated.

Another example includes partial digestion of the nucleic acid with a 5' exonuclease to form a double-stranded nucleic acid with single-stranded 3' overhangs. An adaptor containing a 3' blocking group can be ligated to the 3' ends of double-stranded nucleic acid with 3' overhangs. The double-stranded nucleic acid with 3' overhangs with ligated adaptors can be dehybridized to form single-stranded nucleic acids. An adaptor containing a non-phosphorylated 5' end can be ligated to the 5' end of the single-stranded nucleic acid.

Methods to dephosphorylate nucleic acids, such as the 5' nucleotide of a nucleic acid include contacting a nucleic acid with a phosphatase. Examples of phosphatases include calf intestinal phosphatase, shrimp alkaline phosphatase, Antarctic phosphatase, and APEX alkaline phosphatase (Epicentre).

Methods to ligate nucleic acids include contacting nucleic acids with a ligase. Examples of ligases include T4 RNA ligase 1, T4 RNA ligase 2, RtcB ligase, Methanobacterium RNA ligase, and TS2126 RNA ligase (CIRCLIGASE).

Methods to phosphorylate nucleic acids, such as the 5' nucleotide of a nucleic acid include contacting a nucleic acid with a kinase. Examples of kinases include T4 polynucleotide kinase.

Aspects provided herein relate to preparing nucleic acids libraries in a contiguity particle, such that the nucleic acid library is prepared in a single reaction volume.

Aspects of the systems and methods provided herein include kits, containing any one or more of the hydrogel polymers, crosslinkers, or microfluidic devices for preparing contiguity particles, and further including components useful for processing of the genetic material, including reagents for cell lysis, and nucleic acid amplification and sequencing, or for nucleic acid library preparation, including lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations as described herein, and as used for the respective processing of genetic material.

### EXAMPLES

### Example 1-Preparation of Contiguity Particles

The following example demonstrates an aspect of preparing contiguity particles associated with transposome using a microfluidic droplet generator.

Samples containing cells stored at -80°C were thawed at room temperature. 100 µL of each sample was transferred to a sterile 1.7 mL tube, and the sample was washed once with 1 mL 0.85% NaCl. The sample was pelleted and wash solution was removed. The cell pellet was mixed with a hydrogel solution to resuspend the cells in the hydrogel solution.

To generate contiguity particles of a uniform size distribution, microfluidic droplet generators were used, such as the generator illustrated in FIG. 1. The solution containing a hydrogel polymer and a cell was introduced into a first channel of the microfluidic droplet generator. Mineral oil, used as a spacer oil, was added to a second channel, and a crosslinker was added to a third channel. Upon contacting the crosslinker in the third channel, the hydrogel instantaneously formed contiguity particles associated with a transposome. The type of crosslinking oil was selected to tune the rapidity of crosslinking, including a slow crosslinker or an instantaneous crosslinker, as shown in Table 1:

**Table 1: Crosslinking Chemistries**

| **Contiguity Particle Type** | **Crosslinkers** | **Crosslinking Time** | **Size Homogeneity** | **Bead Curing** |
|---|---|---|---|---|
| Slow Crosslinker | PEG-epoxide + Peg-amine | >12 hours | Good | Complete |
| | PEG-dithiol + PEG-acrylate | 4 hours | Average | Incomplete |
| Instantaneous Crosslinker | Dithiol Oil + PEG-maleimide | Instantaneous | Good | Complete |
| | PEG epoxide + amine oil | Instantaneous | Good | Complete |

### Example 2-Co-Assavs Performed on Contiguity Particles

The following example demonstrates exemplary assays performed on contiguity particles from Example 1, including SCI-seq, ATAC-seq, combinatorial indexing, and single cell whole genome amplification.

Contiguity particles from Example 1 were obtained and deposited onto a plate having wells, such that a single contiguity particle was deposited into a single well. Cells were lysed by introducing a lysis buffer, following by wash, thereby extracting nucleic acids from the cells. The contiguity particles with the lysed cells were then exposed to a series of assays as described below.

Indexed transposomes (TSM) were used to tagment genomic DNA, generating ATAC-seq fragments. After proteinase/SDS treatment, the oligoT with the same index as TSM was added to each well to initiate cDNA synthesis by reverse transcription (RT). The PCR adapter on the other end was introduced by randomer extension. This generated Index 1. Contiguity particles from each well were pooled together then split into an indexed PCR plate to generate Index 2. This 2-tier indexing can be scaled up to 150,000 (384x384) cells; The final library generated was a mixture of ATAC-seq and RNA-seq, in which the gDNA and cDNA from the same cell was grouped by the same index, and the oligoT-UMI pattern served as an internal marker to distinguish RNA signal from ATAC signal.

In addition, random extension was also used for full length RNA-seq. In this case the indices of TSM were different from the indices of the randomers. The 1-to-1 matching between these 2 indices set helped identify the reads from a single cell and differentiated DNA and RNA signals. UMI was also applied in this method to improve the accuracy of reads analysis.

A 3-tiered combinatorial indexing assay was also performed using two rounds of indexed splint-ligation and one round of indexed PCR. In this method, TSM and oligoT are universal, both containing a splint1 fragment, which enabled the index addition by splint-ligation. Three-tier indexing achieved up to 1 million cell throughput (96x96x96). Another 3-tier combinatorial indexing for ATAC-seq was performed using indexed TSMs to increase cell throughput. Rather than using a universal TSM, TSMs contained a unique index on its B7G and A7G side. Two different splints were used to attach indexed adapters required for indexed PCR. The indexing included the following components: B15 adaptor sequence (GTCTCGTGGGCTCGG; SEQ ID NO: 5), N6, and Link1, together forming a B15_N6_Link1 sequence (GTCTCGTGGGCTCGGNNNNNNGACTTGTC; SEQ ID NO: 11); a Phos_Link2, A7G sequence (TGGTAGAGAGGGTG; SEQ ID NO: 9), and ME sequence (AGATGTGTATAAGAGACAG; SEQ ID NO: 7), together forming a Phos _Link2_A7G_ME sequence (TAGAGCATNNNNNNTGGTAGAGAGGGTGAGATGTGTATAAGAGACAG; SEQ ID NO: 12); an A14 adaptor sequence (TCGTCGGCAGCGTC; SEQ ID NO: 6), N6, and Link1, together forming an A14_N6_Link1 sequence (TCGTCGGCAGCGTCNNNNNNGTAATCAC; SEQ ID NO: 13); and a Phos_Link2, B7G (TACTACTCACCTCCC; SEQ ID NO: 10), and ME sequence (AGATGTGTATAAGAGACAG; SEQ ID NO: 7), together forming a Phos _Link2_B7G_ME sequence (CATCATCCNNNNNNTACTACTCACCTCCCAGATGTGTATAAGAGACAG; SEQ ID NO: 14). This example also provides an ME complementary sequence (TCTACACACATTCTCTGTC; SEQ ID NO: 8), a Splint 1 sequence (ATGCTCTAGACAAGT; SEQ ID NO: 15), and a Splint 2 sequence (GGATGATGGTGATTA; SEQ ID NO: 16). In the sequences, N is A, C, T, or G.

Single cell whole genome amplification was also carried out using the contiguity particles. This was done using Indexed T7 transposition of contiguity particles in individual wells followed by pooling and extension via T7 in vitro transcription (IVT) linear amplification. The beads were separated again for indexed random extension, pooled and split again for a final indexed PCR.

The contiguity particles showed efficient tagmentation when they were targeted by FAM labeled transposomes. The nuclei were stained with Hoechst (DNA stain with blue emission) while the transposed nuclei were stained fluorescent green with FAM (fluorescent dye). Lysing the cells with SDS increased the background fluorescence of contiguity particles while there was no signal seen from contiguity particles without any cells. The results demonstrate that an ATAC-seq library may be generated for nucleic acid molecules associated with contiguity particles with only small portion of leakage of short fragments from tagmentation.

### Example 3-Nucleic Acid Library Preparation in Contiguity Particles

The following example demonstrates a method for nucleic acid preparation using contiguity particles.

Contiguity particles as prepared in Example 1 were obtained. The contiguity particles (CPs) were loaded on a 45 µm cell strainer and multiple washes with PBS or Tris-Cl were performed to remove any non-encapsulated cells. One advantage of encapsulating a cell in a contiguity particle is an improved ability to handle and process them. One simple way to do this is through use of spin columns or filter plates. The pore size of the filter may be smaller than the bead diameter. Examples of filter plates include but not limited to Millipore's MultiScreen-Mesh Filter Plates with 20, 40, and 60 µm pore size, Millipore's MultiScreen Migration Invasion and Chemotaxis Filter Plate with 8.0 µm pores, or Pall's AcroPrep Advance 96-Well Filter Plates for Aqueous Filtration with 30-40 µm pores. With these filter plates, bead encapsulated cells were easily separated from solution, allowing multiple buffer exchanges.

The washed beads were suspended in buffer and removed from the filter. To estimate the final concentration of beads, efficiency of cell loading, and to ensure that no non-encapsulated cells remained, an aliquot of the beads was visualized under microscope.

To perform Nextera tagmentation, Millipore's 20 µm Nylon MultiScreen-Mesh Filter Plates were used. To limit adhesion of beads to the filter, they were pre-wet with Pluronic F-127. After centrifugation of the plate for 30s @ 500g, buffer flowed through the filter will maintaining the beads. The beads were washed twice with 200 µL of Tris-Cl buffer, then suspended in lysis buffer (0.1% SDS). Beads were incubated in lysis buffer for Imin before removal by centrifugation. To remove residual lysis buffer, two additional 200 µL Tris-Cl washes were performed. Next, cells were suspended in 45 µL of 1× Tagmentation buffer by pipetting up and down, then transferred to a strip tube. To 45 µL of beads, 5 µL of Tagmentation DNA Enzyme (TDE, Illumina Inc.) was added and incubation in a thermal cycler was performed for 1hr @ RT, 30 minutes at 55°C. Alternatively, tagmentation could be performed on the filter plate by suspending bead-encapsulated cells in the tagmentation master mix and incubating on a heat block. After tagmentation, an aliquot of beads was stained with Hoechst dye and visualized under microscope. Visualization confirmed that DNA remains in the bead.

Tagmented beads (25 µL) were PCR amplified using Illumina's Nextera PCR MM (NPM, Illumina) and PCR primers. To the master PCR mix, 0.1% SDS was added to remove Tn5 bound to DNA. Pre-incubation was performed at 75°C to help Tn5 removal in presence of SDS. Eleven PCR cycles were performed to generate a final library. Following PCR, libraries were purified using 0.9x SPRI, quantified using dsDNA qubit and/or BioAnalyzer, and sequenced on the MiSeq and/or NextSeq.

The method described in this example may be scaled up to perform single cell sequencing using an approach similar to sciSEQ (Vitak et al. Nat Meth. 2017;14:302-308). For example, use of a 96-well filter plate to perform 96 indexed-tagmentation reactions simultaneously may be performed in a scale up process. After beads are added to the plate, successive buffers are added, then removed, by centrifugation or vacuum. After tagmentation, beads are collected from the filter and pooled. Pooled beads are redistributed in a second 96-well PCR plate for multiplexed PCR. In this dual level indexing scheme (Tagmentation and PCR indexing) all DNA fragments from a single bead-encapsulated cell receive the same barcode that can be deconvoluted to reconstruct the cell's genome.

The steps outlined in this example are amenable to automation on liquid handling platforms by addition of a vacuum manifold, such as Millipore's MultiScreen HTS Vacuum Manifold or Orochem's 96-well Plate Vacuum Manifold. These vacuum manifolds can be added to many liquid handling platforms, including the Biomek FX, the Microlab Star, the Tecan Genesis, etc. Tagmentation could be automated by transferring the filter plate to a thermal block. The plate is then transferred back to the vacuum manifold for post tagmentation washes.

### Example 4-Long Read Indexing

The following example demonstrates a method chromosome level phasing using the long read indexing methods and systems provided herein.

Contiguity particles (also referred to herein as contiguity beads) having transposomes associated therewith were prepared, and subjected to linked long read methods described herein, as analyzed on human leukocyte antigen (HLA). In addition to the contiguity particles, also prepared were primer beads, each primer bead having an adapter, a barcode, and a primer. The contiguity beads and the primer beads were partitioned together within droplets (one contiguity bead and one primer bead per droplet) together with a solution primer mixed that included adapter and primers. On bead tagmentation, amplification, and indexing was performed with the partitioned droplets in order to index a bead pool of greater than 900,000 barcodes, each barcode relatively equally represented.

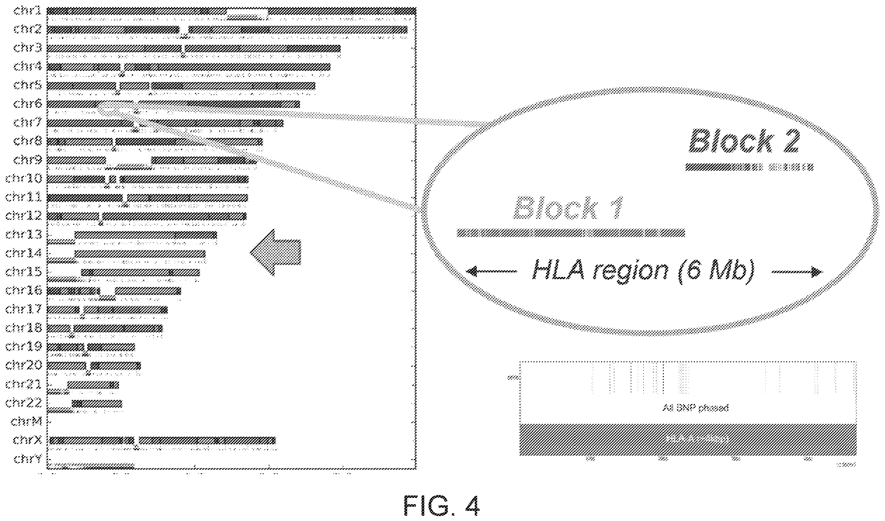

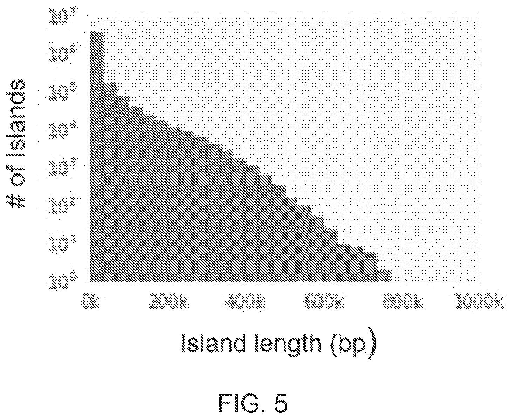

As depicted in FIG. 4, chromosome level phasing was obtained for HLA. Using this assay, up to 26 Mb chromosome level phasing was achieved, with only 1 in 50 Mb long switch error, and covering more than 99% of SNPs. The analysis required a single day of assays (over a period of 5.7 hours), compared to the typical 2 days assay as required for 10X sequencing. Further, as shown in FIG. 5, the number of islands compared to island length using the long read indexing methods described herein exhibits high DNA quality on phasing metrics.

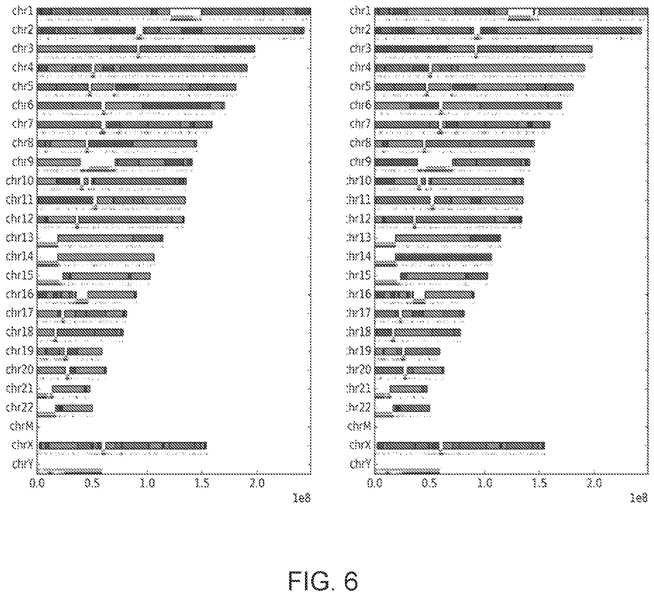

FIG. 6 depicts results of variant calling and phasing blocks using the long read indexing methods described herein (left) compared to 10X sequencing (right). These results indicate that the methods provided herein result in a mean coverage that exceeds 10X sequencing and have greater INDEL precision.

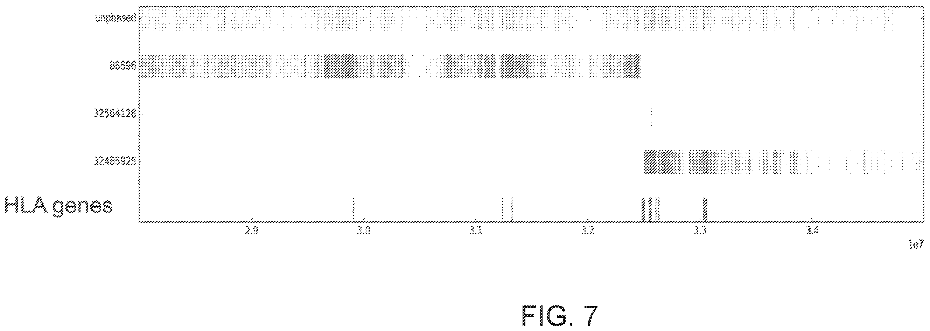

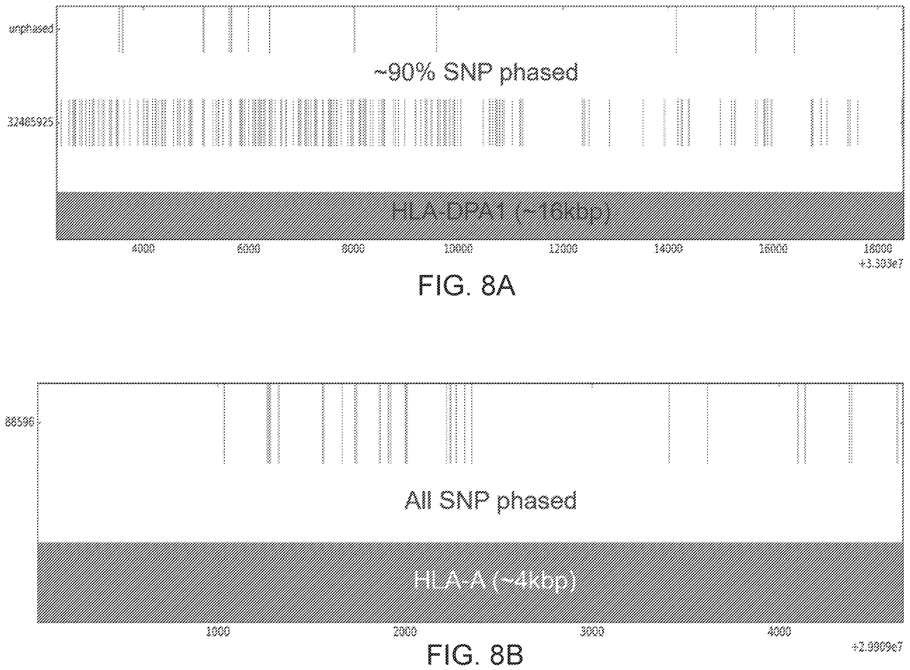

Finally, as shown in FIG. 7 and FIGs. 8A and 8B, the methods of long read indexing methods described herein performed on a HLA region (FIG. 7), HLA-DPA1 (FIG. 8A), and HLA-A (FIG. 8B). The detailed results of the long indexed read methods as described herein compared to 10X sequencing is provided in Table 2:

**Table 2: Detailed Indexed Read Results**

| **Metric** | **Indexed Read Method** | **10X** |
|---|---|---|
| Data Yield (G) | 127 (23X) | 150 (30X |
| Percent Unique Decoded Reads | 79 | 68.1-95.1 |
| Average Insert Size (bp) | 175 | 237-446 |
| Island Coverage | 11.8% | 8.7-14% |
| N50 Island Length | 100k | 38k-58k |
| Phased Het SNPs (%) | 99.1 | 97.2-99.7 |
| Block N50 (Mb) | 26 | 2-15 |
| Longest Block (Mb) | 86.3 | 39.9 |
| Short Switch SNP (%) | 0.12 | 0.15-0.17 |
| Long Switch (%) | 0.002 | 0.002-0.005 |
| Active Barcodes | >900K | >200K |
| DNA per Partition (Kb) | 6000 | 500 |

The aspects, examples, and figures described herein provide compositions, methods, and systems for retaining genetic material in physically confined space during the process from lysis to library generation. Some aspects provide libraries originated from single long DNA molecule or a single cell to be released on a surface of a flow cell in confined space. Once the library from a single DNA molecule or single cell in the individual compartments are released to the surface of the flow cell, the library from each compartment gets seeded at close proximity to each other.

The term "comprising" as used herein is synonymous with "including," "containing," or "characterized by," and is inclusive or open-ended and does not exclude additional, unrecited elements or method steps.

## Claims

1. A system for nucleic acid indexed amplification, comprising:
a plurality of contiguity beads, each contiguity bead associated with a transposome, and comprising a bead-bound nucleic acid molecule;
an indexed primer pool comprising:
a plurality of primer beads, each primer bead comprising an adapter, a barcode, and a primer; and
a solution primer;
wherein the contiguity beads and primer beads are partitioned together within droplets; and
a detector for obtaining sequencing data.

2. The system of claim 1, wherein the contiguity beads and/or the primer beads are hydrogel beads comprising a hydrogel polymer and a crosslinker, optionally,
wherein the hydrogel polymer comprises polyethylene glycol (PEG)-thiol/PEG-acrylate, acrylamide/N,N'-bis(acryloyl)cystamine (BACy), PEG/polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, or collagen, and optionally, wherein the crosslinker comprises bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate.

3. The system of claim 1, wherein the nucleic acid is a DNA molecule of 50,000 base pairs or greater.

4. The system of claim 1, wherein the primer is a P5 primer,
optionally, wherein the solution primer comprises adapters and primers, and optionally
wherein the solution primer comprises B15 adapters and P7 primers.

5. The system of claim 1, wherein the transposome comprises transposase and transposon.

6. A flow cell device for nucleic acid indexed amplification, comprising:
a solid support comprising a plurality of partitioned droplets comprising:
a contiguity bead associated with a transposomes, and comprising a bead-bound nucleic acid molecule; and
a primer bead comprising an adapter, a barcode, and a primer;
wherein the plurality of partitioned droplets are distributed along a surface of the solid support.

7. The flow cell device of claim 6, wherein the solid support is functionalized with a surface polymer
and, optionally, wherein the surface polymer is poly(N-(5-azidoacetamidylpentyl) acrylamide-co-acrylamide) (PAZAM) or silane free acrylamide (SFA).

8. The flow cell device of claim 6, wherein the flow cell comprises a patterned surface,
optionally, wherein the patterned surface comprises wells,
optionally, wherein the wells are from about 10 µm to about 50 µm in diameter, such as 10 µm, 15 µm, 20 µm, 25 µm, 30 µm, 35 µm, 40 µm, 45 µm, or 50 µm in diameter, or within a range defined by any two of the aforementioned values, and wherein the wells are about 0.5 µm to about 1 µm in depth, such as 0.5 µm, 0.6 µm, 0.7 µm, 0.8 µm, 0.9 µm, or 1 µm in depth, or within a range defined by any two of the aforementioned values, optionally
wherein the wells are comprised of hydrophobic material, and
optionally wherein the hydrophobic material comprises an amorphous fluoropolymer, such as CYTOP, Fluoropel^{®}, or Teflon^{®}.

9. The flow cell device of claim 6, wherein the nucleic acid is a DNA molecule of 50,000 base pairs or greater.

10. The flow cell device of claim 6, wherein the transposome comprises transposase and transposon.

11. A method of nucleic acid indexing comprising:
generating a plurality of contiguity beads for on bead tagmentation, each bead linked to a transposome, and comprising a bead-bound nucleic acid molecule
performing a tagmentation reaction on the nucleic acid molecule;
generating a plurality of primer beads, each primer bead comprising an adapter, a barcode, and a primer;
partitioning the contiguity beads and the primer beads together within droplets with a solution primer;
amplifying nucleic acid molecule within the partitioned droplets; and
indexing the nucleic acid molecule in each droplet.

12. The method of claim 11, wherein the nucleic acid is a DNA molecule of 50,000 base pairs or greater.

13. The method of claim 11, further comprising performing nucleic acid amplification on nucleic acid molecule prior to performing the tagmentation reaction.

14. The method of claim 13, wherein the amplification reaction comprises multiple displacement amplification (MDA),
optionally, wherein the tagmentation reaction comprises contacting the nucleic acid with a transposase mixture comprising adapter sequences and transposomes, and
optionally, wherein the indexing is performed by polymerase chain reaction (PCR).

15. The method of claim 11, wherein the droplets are partitioned into more than 900,000 different indexed PCR compartments,
optionally, further comprising partitioning the droplets on a solid support, and
optionally, wherein the solid support is a flow cell device.

## Patentansprüche

1. System zur indexierten Nukleinsäure-Amplifikation, das Folgendes aufweist:
eine Vielzahl von Kontiguitätskügelchen, wobei jedes Kontiguitätskügelchen mit einem Transposom assoziiert ist und ein kügelchengebundenes Nukleinsäuremolekül aufweist;
einen Pool indexierter Primer, der Folgendes aufweist:
eine Vielzahl von Primerkügelchen, wobei jedes Primerkügelchen einen Adapter, einen Barcode und einen Primer aufweist; und
einen Lösungssprimer;
wobei die Kontiguitätskügelchen und Primerkügelchen zusammen in Tröpfchen partitioniert sind; und
einen Detektor zur Gewinnung von Sequenzierungsdaten.

2. System nach Anspruch 1, wobei die Kontiguitätskügelchen und/oder die Primerkügelchen Hydrogelkügelchen sind, die ein Hydrogelpolymer und einen Vernetzer aufweisen,
wobei das Hydrogelpolymer optional Polyethylenglykol (PEG)-Thiol/PEG-Acrylat, Acrylamid/NN'-Bis(acryloyl)cystamin (BACy), PEG/Polypropylenoxid (PPO), Polyacrylsäure, Poly(hydroxyethylmethacrylat) (PHEMA), Poly(methylmethacrylat) (PMMA), Poly(N-isopropylacrylamid) (PNIPAAm), Polymilchsäure (PLA), Polylactidco-glycolid (PLGA), Polycaprolacton (PCL), Poly(vinylsulfonsäure) (PVSA), Poly(L-asparaginsäure), Poly(L-glutaminsäure), Polylysin, Agar, Agarose, Alginate, Heparin, Alginsäuresulfat, Dextransulfat, Hyaluronan, Pektin, Carrageenan, Gelatine, Chitosan, Zellulose oder Kollagen aufweist; und optional,
wobei der Vernetzer Bisacrylamid, Diacrylat, Diallylamin, Triallylamin, Divinylsulfon, Diethylenglycoldiallylether, Ethylenglycoldiacrylat, Polymethylenglycoldiacrylat, Polyethylenglycoldiacrylat, Trimethylopropantrimethacrylat, ethoxyliertes Trimethyloltriacrylat oder ethoxyliertes Pentaerythritoltetraacrylat aufweist.

3. System nach Anspruch 1, wobei die Nukleinsäure ein DNA-Molekül mit 50.000 Basenpaaren oder mehr ist.

4. System nach Anspruch 1, wobei der Primer ein P5-Primer ist,
wobei der Lösungssprimer optional Adapter und Primer aufweist, und optional,
wobei der Lösungsprimer B15-Adapter und P7-Primer aufweist.

5. System nach Anspruch 1, wobei das Transposom eine Transposase und ein Transposon aufweist.

6. Flusszellenvorrichtung zur indexierten Nukleinsäure-Amplifikation, die Folgendes aufweist:
einen Festkörper, der eine Vielzahl partitionierter Tröpfchen aufweist, der Folgendes aufweist:
ein Kontiguitätskügelchen, das mit einem Transposom assoziiert ist und ein kügelchengebundenes Nukleinsäuremolekül aufweist; und
ein Primerkügelchen, das einen Adapter, einen Barcode und einen Primer aufweist;
wobei die Vielzahl der partitionierten Tröpfchen entlang einer Oberfläche des Festkörpers verteilt ist.

7. Flusszellenvorrichtung nach Anspruch 6, wobei der Festkörper mit einem Oberflächenpolymer funktionalisiert ist,
und optional, wobei das Oberflächenpolymer Poly(N-(5-azidoacetamidylpentyl)-acrylamid-co-acrylamid) (PAZAM) oder silanfreies Acrylamid (SFA) ist.

8. Flusszellenvorrichtung nach Anspruch 6, wobei die Flusszelle eine strukturierte Oberfläche aufweist,
optional, wobei die strukturierte Oberfläche Vertiefungen aufweist,
optional, wobei die Vertiefungen einen Durchmesser von etwa 10 µm bis etwa 50 µm haben, wie z.B. 10 µm, 15 µm, 20 µm, 25 µm, 30 µm, 35 µm, 40 µm, 45 µm oder 50 µm im Durchmesser oder innerhalb eines Bereichs, der durch zwei der oben genannten Werte definiert ist, und wobei die Vertiefungen eine Tiefe von etwa 0,5 µm bis etwa 1 µm aufweisen, wie z.B. 0,5 µm, 0,6 µm, 0,7 µm, 0,8 µm, 0,9 µm oder 1 µm in der Tiefe oder innerhalb eines Bereichs, der durch zwei der oben genannten Werte definiert ist; optional
wobei die Vertiefungen hydrophobes Material aufweisen, und
optional wobei das hydrophobe Material ein amorphes Fluorpolymer wie CYTOP, Fluoropel^{®} oder Teflon^{®} aufweist.

9. Flusszellenvorrichtung nach Anspruch 6, wobei die Nukleinsäure ein DNA-Molekül mit 50.000 Basenpaaren oder mehr ist.

10. Flusszellenvorrichtung nach Anspruch 6, wobei das Transposom eine Transposase und ein Transposon aufweist.

11. Verfahren zur Indexierung von Nukleinsäuren, das Folgendes aufweist:
Erzeugen einer Vielzahl von Kontiguitätskügelchen für die Tagmentierung an Kügelchen, wobei jedes Kügelchen mit einem Transposom verbunden ist und ein kügelchengebundenes Nukleinsäuremolekül aufweist;
Durchführen einer Tagmentierungsreaktion an dem Nukleinsäuremolekül;
Erzeugen einer Vielzahl von Primerkügelchen, wobei jedes Primerkügelchen einen Adapter, einen Barcode und einen Primer aufweist;
Partitionieren der Kontiguitätskügelchen und der Primerkügelchen zusammen in Tröpfchen mit einem Lösungssprimer;
Amplifizieren des Nukleinsäuremoleküls innerhalb der partitionierten Tröpfchen; und
Indexieren des Nukleinsäuremoleküls in jedem Tröpfchen.

12. Verfahren nach Anspruch 11, wobei die Nukleinsäure ein DNA-Molekül mit 50.000 Basenpaaren oder mehr ist.

13. Verfahren nach Anspruch 11, das ferner die Durchführung einer Nukleinsäure-Amplifikation an dem Nukleinsäuremolekül vor der Durchführung der Tagmentierungsreaktion aufweist.

14. Verfahren nach Anspruch 13, wobei die Amplifikationsreaktion eine multiple displacement amplification (MDA) aufweist,
optional, wobei die Tagmentierungsreaktion das Kontaktieren der Nukleinsäure mit einer Transposasemischung aufweist, die Adaptersequenzen und Transposome aufweist, und
optional, wobei das Indexieren durch Polymerase-Kettenreaktion (PCR) durchgeführt wird.

15. Verfahren nach Anspruch 11, wobei die Tröpfchen in mehr als 900.000 verschiedene indexierte PCR-Kompartimente partitioniert werden,
optional, ferner das Partitionieren der Tröpfchen auf einem Festkörper aufweist, und
optional, wobei der Festkörper eine Flusszellenvorrichtung ist.

## Revendications

1. Système d'amplification indexée d'acide nucléique, comprenant :
une pluralité de billes de contiguïté, chaque bille de contiguïté étant associée à un transposome et comprenant une molécule d'acide nucléique liée à la bille ;
un ensemble d'amorces indexées comprenant :
une pluralité de billes d'amorce, chaque bille d'amorce comprenant un adaptateur, un code-barres et une amorce ; et
une amorce de solution ;
dans lequel les billes de contiguïté et les billes d'amorce sont partitionnées ensemble dans des gouttelettes ; et
un détecteur pour obtenir des données de séquençage.

2. Système de la revendication 1, dans lequel les billes de contiguïté et/ou les billes d'amorce sont des billes d'hydrogel comprenant un polymère d'hydrogel et un agent de réticulation, éventuellement,
dans lequel le polymère d'hydrogel comprend le polyéthylène glycol (PEG)-thiol/PEG-acrylate, l'acrylamide/N,N'-bis(acryloyl)cystamine (BACy), le PEG/oxyde de polypropylène (PPO), l'acide polyacrylique, le poly(méthacrylate d'hydroxyéthyle) (PHEMA), le poly(méthacrylate de méthyle) (PMMA), le poly(N-isopropylacrylamide) (PNIPAAm), le poly(acide lactique) (PLA), le poly(acide lactique-co-glycolique) (PLGA), la polycaprolactone (PCL), le poly(acide vinylsulfonique) (PVSA), le poly(acide L-aspartique), le poly(acide L-glutamique), la polylysine, l'agar, l'agarose, l'alginate, l'héparine, le sulfate d'alginate, le sulfate de dextrane, l'hyaluronane, la pectine, le carraghénane, la gélatine, le chitosane, la cellulose ou le collagène, et éventuellement, dans lequel l'agent de réticulation comprend le bisacrylamide, le diacrylate, la diallylamine, la triallylamine, la divinylsulfone, l'éther diallylique de diéthylèneglycol, le diacrylate d'éthylèneglycol, le diacrylate de polyméthylèneglycol, le diacrylate de polyéthylèneglycol, le triméthacrylate de triméthylopropane, le triacrylate de triméthylol éthoxylé ou le tétraacrylate de pentaérythritol éthoxylé.

3. Système de la revendication 1, dans lequel l'acide nucléique est une molécule d'ADN de 50000 paires de bases ou plus.

4. Système de la revendication 1, dans lequel l'amorce est une amorce P5,
éventuellement, dans lequel l'amorce de solution comprend des adaptateurs et des amorces, et éventuellement,
dans lequel l'amorce de solution comprend des adaptateurs B15 et des amorces P7.

5. Système de la revendication 1, dans lequel le transposome comprend une transposase et un transposon.

6. Dispositif à cellules d'écoulement pour l'amplification indexée d'acide nucléique, comprenant :
un support solide comprenant une pluralité de gouttelettes partitionnées comprenant :
une bille de contiguïté associée à un transposome, et comprenant une molécule d'acide nucléique liée à la bille ; et
une bille d'amorce comprenant un adaptateur, un code-barres et une amorce ;
dans lequel la pluralité de gouttelettes partitionnées sont distribuées le long d'une surface du support solide.

7. Dispositif à cellules d'écoulement de la revendication 6, dans lequel le support solide est fonctionnalisé avec un polymère de surface
et, éventuellement, dans lequel le polymère de surface est le poly(N-(5-azidoacétamidylpentyl)acrylamide-co-acrylamide) (PAZAM) ou l'acrylamide sans silane (SFA).

8. Dispositif à cellules d'écoulement de la revendication 6, dans lequel la cellule d'écoulement comprend une surface à motifs,
éventuellement, dans lequel la surface à motifs comprend des puits,
éventuellement, dans lequel les puits ont un diamètre d'environ 10 µm à environ 50 µm, tel que 10 µm, 15 µm, 20 µm, 25 µm, 30 µm, 35 µm 40 µm, 45 µm ou 50 µm, ou dans une plage définie par deux valeurs quelconques des valeurs susmentionnées, et dans lequel les puits ont une profondeur d'environ 0,5 µm à environ 1 µm, telle que 0,5 µm, 0,6 µm, 0,7 µm, 0,8 µm, 0,9 µm ou 1 µm, ou dans une plage définie par deux valeurs quelconques des valeurs susmentionnées, éventuellement
dans lequel les puits sont constitués d'un matériau hydrophobe, et
éventuellement dans lequel le matériau hydrophobe comprend un fluoropolymère amorphe, tel que CYTOP, Fluoropel^{®}, ou Téflon^{®}.

9. Dispositif à cellules d'écoulement de la revendication 6, dans lequel l'acide nucléique est une molécule d'ADN de 50000 paires de bases ou plus.

10. Dispositif à cellules d'écoulement de la revendication 6, dans lequel le transposome comprend une transposase et un transposon.

11. Procédé d'indexation d'acide nucléique comprenant :
la génération d'une pluralité de billes de contiguïté pour une tagmentation sur billes, chaque bille étant liée à un transposome et comprenant une molécule d'acide nucléique liée à la bille
la réalisation d'une réaction de tagmentation sur la molécule d'acide nucléique ;
la génération d'une pluralité de billes d'amorce, chaque bille d'amorce comprenant un adaptateur, un code-barres et une amorce ;
le partitionnement des billes de contiguïté et des billes d'amorce ensemble dans des gouttelettes avec une amorce de solution ;
l'amplification de la molécule d'acide nucléique dans les gouttelettes partitionnées ; et
l'indexation de la molécule d'acide nucléique dans chaque gouttelette.

12. Procédé de la revendication 11, dans lequel l'acide nucléique est une molécule d'ADN de 50000 paires de bases ou plus.

13. Procédé de la revendication 11, comprenant en outre la réalisation d'une amplification d'acide nucléique sur la molécule d'acide nucléique avant la réalisation de la réaction de tagmentation.

14. Procédé de la revendication 13, dans lequel la réaction d'amplification comprend une amplification par déplacement multiple (MDA),
éventuellement, dans lequel la réaction de tagmentation comprend la mise en contact de l'acide nucléique avec un mélange de transposases comprenant des séquences d'adaptateur et des transposomes, et
éventuellement, dans lequel l'indexation est réalisée par réaction en chaîne de polymérase (PCR).

15. Procédé de la revendication 11, dans lequel les gouttelettes sont partitionnées en plus de 900000 compartiments PCR indexés différents,
éventuellement, comprenant en outre le partitionnement des gouttelettes sur un support solide, et
éventuellement, dans lequel le support solide est un dispositif à cellules d'écoulement.
